# EUROPEAN PATENT SPECIFICATION

(11) **EP 2 896 685 B1**
(45) Date of publication and mention of the grant of the patent: **22.05.2019**
(21) Application number: 12884600.3
(22) Date of filing: 11.09.2012
(51) Int. Cl.: C12M 3/00, B65D 77/00

(54) **BIOLOGICAL SAMPLE PACKAGING CONTAINER AND BIOLOGICAL SAMPLE CONVEYANCE METHOD USING SAME**
VERPACKUNGSBEHÄLTER FÜR BIOLOGISCHE PROBEN UND TRANSPORTVERFAHREN FÜR BIOLOGISCHE PROBEN DAMIT
RÉCIPIENT D'EMBALLAGE D'ÉCHANTILLON BIOLOGIQUE ET PROCÉDÉ DE TRANSPORT D'ÉCHANTILLON BIOLOGIQUE L'UTILISANT

(43) Date of publication of application: 22.07.2015
(73) Proprietor: Hitachi, Ltd., Tokyo 100-8280 (JP)
(72) Inventor: NOZAKI, Takayuki, Chiyoda-ku Tokyo 100-8280 (JP); KIYAMA, Masaharu, Chiyoda-ku Tokyo 100-8280 (JP); ZHOU, Guangbin, Chiyoda-ku Tokyo 100-8280 (JP); MATSUOKA, Shizu, Chiyoda-ku Tokyo 100-8280 (JP); NAKAJIMA, Ryota, Chiyoda-ku Tokyo 100-8280 (JP); SENDA, Naoko, Chiyoda-ku Tokyo 100-8280 (JP)
(74) Representative: MERH-IP Matias Erny Reichl Hoffmann Patentanwälte PartG mbB
(86) International application number: PCT/JP2012/073132
(87) International publication number: WO 2014/041593

(56) References cited:
- WO-A1-2009/136907
- WO-A1-2012/008368
- JP-A- 2004 154 099
- JP-A- 2006 094 758
- JP-A- 2008 239 168
- JP-A- 2009 031 300
- JP-A- 2009 089 715
- US-A1- 2012 160 714

## Description

### BACKGROUND OF THE INVENTION

### (1) Field of the Invention

The present invention relates to a biological sample packaging container and a biological sample conveyance method, and particularly to a packaging container and a conveyance method thereof suitable for use in a case in which a biological sample manufactured by a cell processing center is accommodated to be conveyed to medical institutions and the like, and is applied to a living body as medical treatment.

### (2) Description of the Related Art

The regenerative medical treatment in which functions of organs and the like are recovered using a biological sample such as regenerated tissue manufactured using cells as raw material is expected as a fundamental medical treatment method for diseases for which there have been no medical treatment methods from the past. A manufacturing process of biological samples such as regenerated tissue used for the regenerative medical treatment is based on a good manufacturing practice (GMP) that is a standard of manufacturing control and quality control of medicinal products and the like. The biological samples are manufactured in a cell processing center (CPC) according to a standard operational procedure (SOP) satisfying the GMP. As the GMP, a law regulated by the Ministry of Health, Labour and Welfare has been enforced in Japan (for example, Ordinance of the Ministry of Health and Welfare No. 179, Notification No. 480 of PAB) . Outside Japan, related laws have been enforced mainly by organizations (for example, the U.S. Food and Drug Administration and the European Commission) in Europe and the United States.

In order to operate the CPC, a large amount of cost and human resources with specialized culture techniques are required. Accordingly, at the stage of industrialization of the regenerative medical treatment, regenerated tissue is manufactured in the CPC as a production base, and the manufacture biological samples are conveyed to medical institutions and research institutions in each area to be possibly used for medical treatment for patients or for research.

As conventional techniques related to the above-described background, there are some reports related to conveyance and a packaging technique of biological samples such as regenerated tissue. Japanese Unexamined Patent Application Publication No. 2008-239168 relates to a multiple sterilized package obtained by multiply packaging chips for pipettes and dishes that are sterilized objects with sterilized bags, and discloses a technique in which the sterilized objects are sequentially taken out of the package in accordance with the space cleanliness in a facility after conveyance to keep the cleanliness of the inner sterilized objects.

Japanese Unexamined Patent Application Publication No. 2004-154099 discloses a culture container accommodation box in which an open-system culture container with the inside unsealed can be aseptically stored. The culture container accommodation box is provided with a filter that allows only gas to pass through and into which bacteria and particles do not enter from the outside. Using the box, the open-system culture container is stored in a cell preparation room of a cell processing center, and thus the box can be carried out of the cell processing center in a state where the cleanliness is kept.

WO 2012/008368 A1 concerns a cell culture vessel and a cell culture device. US 2012/160714 A1 refers to a container assembly and a method for containing biological graft. WO 2009/136907 A1 concerns a cell container.
JP 2006-94758 A pertains to a bottle cap structure.

### SUMMARY OF THE INVENTION

In order to reduce the manufacturing cost of the biological sample, it has been required to introduce an automatic culture apparatus that automates some or all of culture processes. Labor-saving and cost reduction are realized not by manually performing the culture process but by the automatic culture apparatus, and mass production can be realized. In addition, an operation by the automatic culture apparatus is constant, and thus the automatic culture apparatus is expected to contribute to the maintained quality of the regenerated tissue obtained after manufacturing.

Given the possibility of introduction of the automatic culture apparatus into the manufacturing process in the future, the manufacturing process by the automatic culture apparatus and the subsequent conveyance process require a technique capable of smoothly shifting the culture container to the conveyance process while keeping the cleanliness.

Further, in the case where the biological sample such as the regenerated tissue manufactured in the cell processing center is stored into the packaging container to be conveyed, it is important to keep the cleanliness.

Specifically, when the conveyed biological sample is used for a living body as medical treatment, the conveyed biological sample needs to keep an excellent state in terms of indexes such as a metabolic function
and a cell survival rate. In particular, it is necessary to keep the cleanliness during the conveyance, and to keep the cleanliness even in a process in which the biological sample is taken out of the container used at the time of the conveyance in the medical treatment. The biological sample manufactured in the CPC passes through a space where the cleanliness is not controlled to an operating room where the medical treatment is conducted. Accordingly, there is a possibility that organisms or particles such as bacteria adhere to the exterior of the container storing the biological sample during the passage. When the medical treatment is conducted, it is necessary to aseptically take out the biological sample in order to avoid biological contamination due to the adhesion of bacteria and the like to the biological sample inside the container. Further, given the possibility of introduction of the automatic culture apparatus into the manufacturing process in the future, the manufacturing process by the automatic culture apparatus and the subsequent conveyance process require a technique of smoothly switching the process.

In the method described in Japanese Unexamined Patent Application Publication No. 2008-239168, the multiply-packaged multiple sterilized package is used at the time of the conveyance. Thus, there is a low risk that the content in the sterilized package is contaminated from the outside, and the package can be conveyed while the cleanliness is kept. However, in the case where the culture is performed by the automatic culture apparatus using the open-system culture container storing the biological sample and the culture medium soaked with the same and then the open-system culture container is conveyed in the subsequent conveyance process, the culture medium is leaked from the open-system culture container in the sterilized bag during the conveyance, and the biological sample is contacted with the unclean outside through the culture medium at the time of opening the package. As a result, there is a risk of biological contamination. Therefore, it is necessary to provide a mechanism by which the culture medium is not leaked from the culture container during the conveyance.

Further, the conveyed biological sample needs to be observed and inspected to check the quality thereof before use for medical treatment such as transplantation. In the case where the cell processing center and the operating room are located on the same premise and the conveyance time is short, checking the conveyed biological sample is not necessarily needed. However, in the case where the both are located on different premises and long-time conveyance is required, it is necessary to check the quality of the conveyed biological sample. In addition, the method thereof needs to be a non-invasive method. In the method described in Japanese Unexamined Patent Application Publication No. 2008-239168, in the case where the quality of the conveyed biological sample is checked, the container storing the biological sample is taken out by opening the multiply-packaged sterilized bag, and the cells are non-invasively observed using a phase-contrast microscope or the like. In this case, if there is no cell processing center that can keep the aseptic state in medical institutions and research institutions that are destinations, the biological sample is observed by opening the sterilized bag in an inspection room that is not in an aseptic state. This means that the container storing the biological sample is exposed to a contamination space, and suggests the possibility of contamination due to bacteria and the like adhering to the exterior of the culture container. Even if the culture container is disinfected by ethanol when the culture container is carried into the operating room, all the substances adhering to the culture container cannot be necessarily removed. When the medical treatment such as transplantation is conducted and if the bacteria and particles that could not be removed when the lid of the culture container is opened fall and adhere to the biological sample or enter the culture medium, biological contamination possibly occurs.

In the case where medical institutions and research institutions that are destinations have a cell processing center, the container can be aseptically opened to observe the cells and can be stored into the sterilized bag again. However, it is difficult to demonstrate that the worker conducted the process while keeping the aseptic state. Although the operation is performed in accordance with the SOP, it is difficult to evaluate the sterility after the operation until the medical treatment. Further, owning the cell processing center cannot be easily realized because a large amount of cost and human resources with specialized culture techniques are required.

Further, when the biological sample is taken out of the culture container at the time of transplantation, it is necessary to avoid a leakage of the culture medium in the culture container to the outside. As described above, the container passes through an unclean space during the conveyance, and thus the cleanliness of the exterior of the container storing the biological sample is deteriorated. Further, it is necessary to inspect the biological sample after the conveyance if needed, and thus the cleanliness of the exterior of the culture container is not necessarily kept as similar to the above. Therefore, the leakage of the culture medium when the biological sample is taken out of the culture container means a risk of biological contamination in the biological sample. Accordingly, when the culture container is opened at the time of the medical treatment, it is necessary to avoid a leakage of the culture medium.

The culture container accommodation box described in Japanese Unexamined Patent Application Publication No. 2004-154099 is a container that can aseptically store the open-system culture container. However, if the culture container accommodation box is conveyed in a state where the open-system culture container is stored in the culture container accommodation box, there is a possibility of a leakage of the culture medium from the open-system culture container. Thus, when the biological sample is taken out of the box at the time of the inspection and medical treatment after the conveyance, there is a risk of biological contamination through the leaked culture medium.

As described above, in the case where the biological sample such as regenerated tissue is conveyed, it is necessary to have a function of keeping the cleanliness at the time of the conveyance and when opening the container at the time of the inspection and medical treatment after the conveyance. In order to keep the cleanliness, it is necessary to avoid a leakage of the culture medium from the culture container while the cleanliness is kept during the conveyance. Further, it is necessary to use the biological sample that can be observed while keeping the cleanliness after the conveyance. Further, when the conveyed biological sample is taken out at the time of the medical treatment, a technique of opening the container while preventing a leakage of the culture medium and keeping the clean state is necessary.

Based on the above, an object of the present invention is to provide a biological sample packaging container and a conveyance method thereof by which it is possible to keep the cleanliness of a culture container storing a biological sample at the time of conveyance of the biological sample and before and after the conveyance, and a leakage of a culture medium at the time of a non-invasive inspection and when opening the container can be avoided.

The following is a representative configuration example of the present invention.

The present invention provides a biological sample packaging container including: a sample storage container having a recessed part; a lid member that seals the upper surface of the sample storage container; and at least one gasket that is provided on the rear surface of the lid member or on the upper surface of the sample storage container, wherein: the sample storage container has a function of holding a biological sample therein; the lid member includes a first lid member provided with flow channel tubes that allow liquid and gas to be moved between the sample storage container and the outside, and a second lid member having no flow channel tubes; and the sample storage container is configured in such a manner that the first lid member and the second lid member can be replaced by each other.

According to the biological sample packaging container of the present invention, it is possible to prevent a culture medium from being leaked from the inside during conveyance and when opening the packaging container after the conveyance, and a risk of biological contamination can be suppressed.

### BRIEF DESCRIPTION OF THE DRAWINGS

FIG. 1A is a vertical cross-sectional view for showing a configuration example of a biological sample packaging container;
FIG. 1B is a plan view of the packaging container shown in FIG. 1A;
FIGs. 1C are diagrams obtained by decomposing the packaging container shown in FIG. 1A into constitutional elements;
FIG. 2A is a cross-sectional view for showing another configuration example of the biological sample packaging container for culture;
FIG. 2B is a plan view of the packaging container shown in FIG. 2A;
FIGs. 3A are diagrams each showing an operation of installing and opening a lid part of the packaging container;
FIGs. 3B are diagrams each showing an operation of installing and opening the lid part of the packaging container;
FIG. 4A is a diagram for showing an operation of storing into the packaging container after culture by an automatic culture apparatus or manual culture;
FIG. 4B is a diagram for showing an operation of storing into the packaging container after the culture by the automatic culture apparatus or the manual culture;
FIG. 4C is a diagram for showing an operation of storing into the packaging container after the culture by the automatic culture apparatus or the manual culture;
FIG. 4D is a diagram for showing an operation of storing into the packaging container after the culture by the automatic culture apparatus or the manual culture;
FIG. 5A is a diagram for showing another operation method of storing into the packaging container after the culture by the automatic culture apparatus or the manual culture;
FIG. 5B is a diagram for showing another operation method of storing into the packaging container after the culture by the automatic culture apparatus or the manual culture;
FIG. 5C is a diagram for showing another operation method of storing into the packaging container after the culture by the automatic culture apparatus or the manual culture;
FIG. 6 is a diagram for showing a configuration of a cell conveyance container that conveys a sample container packaged in the packaging container;
FIGs. 7 are diagrams each showing a configuration and function of a heat storage material container stored in the cell conveyance container;
FIGs. 8 are diagrams each showing a configuration and function of a heat storage material container in a comparison example;
FIG. 9 is a diagram for showing a configuration example of the automatic culture apparatus using the packaging container;
FIG. 10 is a diagram for showing an example in which a culture container cultured in the automatic culture apparatus is packaged in the packaging container to be carried out of a CPC;
FIG. 11 is a diagram for showing an example in which the packaging container is carried out of the CPC;
FIG. 12 is a diagram for showing an example in which the packaging container is carried into medical institutions;
FIG. 13A is a vertical cross-sectional view for showing an example of a packaging container;
FIG. 13B is a vertical cross-sectional view for showing another example of the packaging container;
FIG. 13C is a vertical cross-sectional view for showing another example of the packaging container;
FIG. 13D is a vertical cross-sectional view for showing another example of the packaging container;
FIG. 13E is a vertical cross-sectional view for showing another example of the packaging container;
FIG. 14A is a vertical cross-sectional view for showing a packaging container;
FIG. 14B is a plan view for showing the packaging container of FIG. 14A; and
FIG. 15 is a vertical cross-sectional view for showing a packaging container.

### DETAILED DESCRIPTION OF THE DRAWINGS

Representative embodiments of a packaging container include a sample storage container having a recessed part holding therein a sample container, a packaging container that seals the upper surface of the sample storage container, and at least one gasket that is provided on the rear surface of a packaging container lid part. The sample storage container is used to hold the sample container therein at the time of culture of a biological sample and at the time of conveyance of the biological sample. The packaging container lid part includes a first packaging container lid part that has flow channel tubes allowing liquid and gas to be moved between the sample storage container and the outside and that is used at the time of culture of the biological sample, and a second packaging container lid part that has no flow channel tubes and that is used at the time of conveyance of the biological sample.

Further, the packaging container includes a packaging container main body part that holds the sample container having the biological sample therein, and the packaging container lid parts (the first packaging container lid part and the second packaging container lid part) that seal the packaging container main body part. The packaging container main body part or the packaging container lid part has a first gasket that suppresses a risk of biological contamination by suppressing the movement of a culture medium during the conveyance and when opening the packaging container, and a second gasket that suppresses the movement of gas and liquid when being crimped, keeps the cleanliness inside the packaging container by preventing bacteria and the like from entering from the outside of the packaging container, and keeps the inner airtightness even under reduced pressure in the case of air transportation by aircraft. These first and second gaskets suppress bacteria from entering from the outside of the packaging container. In addition, the movement of the culture medium is suppressed at the time of conveyance and opening, and as a result, a risk of biological contamination is suppressed.

Further, the packaging container main body part allows the second packaging container lid part used for conveyance to be replaced by the first packaging container lid part having the flow channel tubes that allow liquid and gas to be moved to/from the outside. Using the biological sample packaging container having such lid members for culture, culture can be performed by an automatic culture apparatus that can perform all or any one of culture medium replacement in an airtight state, a microscope observation, and automatic culture. Specifically, after the culture by the automatic culture apparatus using the first packaging container lid part, the lid member attached at the time of automatic culture is replaced by the second packaging container lid part for conveyance to package the packaging container main body part, and the container can be conveyed while keeping the original state of the packaging container main body part and keeping the cleanliness of the inner sterilized objects.

It should be noted that each of the terms "sample storage container", "sample container", "culture container", and "container" means a "culture container" in the specification and claims. Hereinafter, the terms "sample container", "culture container", and "container" are possibly used together with or instead of the "culture container" to easily understand the explanation.

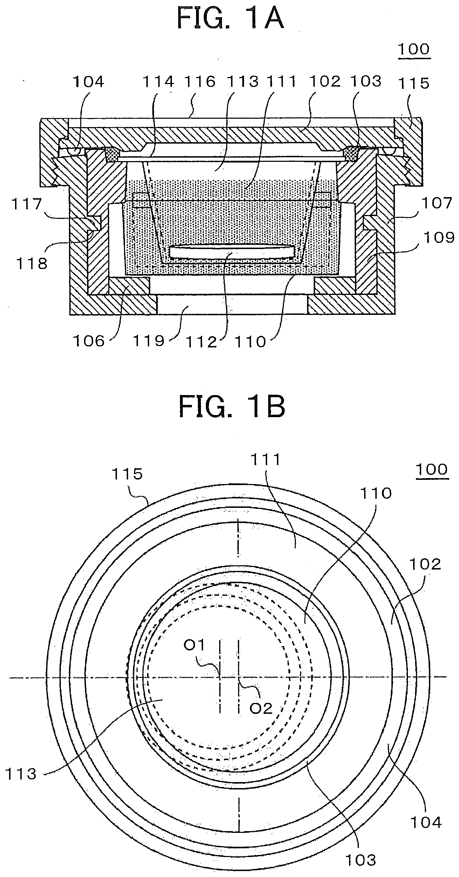

A first packaging container will be described with reference to FIG. 1A to FIG. 12.

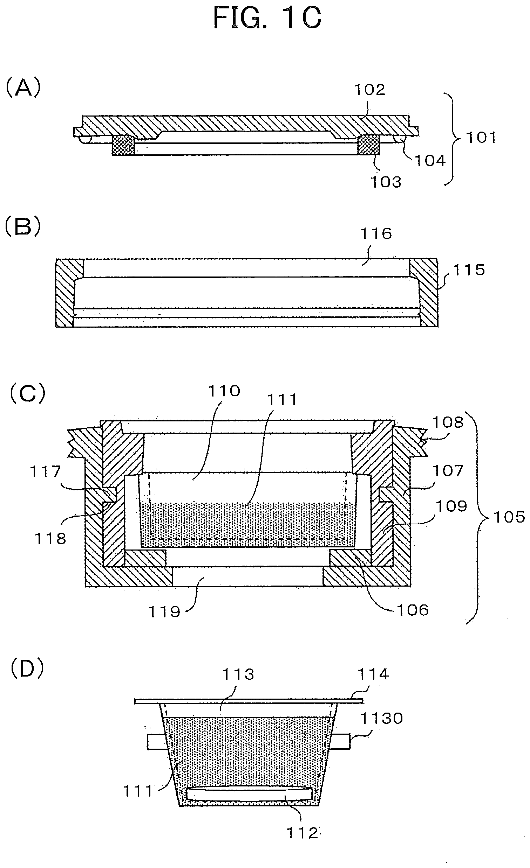

First, basic constitutional elements of a packaging container for conveyance that stores a sample container (culture container) having a biological sample therein according to the first embodiment will be described using FIG. 1A to FIGs. 1C. The sample container having the biological sample therein such as regenerated tissue is packaged using a packaging container main body part and a packaging container lid part. In this case, FIG. 1A shows a vertical cross-sectional view of a biological sample packaging container 100 for conveyance used when regenerated tissue cultured using an insertion-type culture container is conveyed, FIG. 1B shows a plan view thereof, and FIGs. 1C show diagrams decomposed into constitutional elements. The biological sample packaging container 100 is a packaging container holding therein sample containers (a sample container 110 and an insertion-type culture container 113) having a biological sample, and includes a sample storage container (a packaging container main body part 105 (see FIGs. 1C)) provided with a recessed part holding therein the sample containers, a second lid member (a second packaging container lid part 101 (see FIGs. 1C)) that seals the upper surface of the sample storage container, and a screw ring part 115. The second packaging container lid part 101 includes a circular first gasket 103 that abuts on the upper-end surface of the recessed part when sealing the sample storage container and a circular second gasket 104 that abuts on the upper surface of the side wall of the sample storage container. The first gasket 103 and the second gasket 104 are fixed to the rear surface of a transparent packaging container lid part main body 102. The first gasket 103 is longer than the second gasket 104 in the direction where the packaging container main body part 105 is sealed with the packaging container lid part 102. It should be noted that flow channel tubes and the like are not provided on the top surface side of the packaging container lid part main body 102 because the packaging container is used for conveyance.

The sample storage container (the packaging container main body part 105) is configured using a cup-like packaging container main body part bottom section 107 having a screw structure 108 at an upper-end outer edge and a substantially-cylindrical packaging container main body part holding section 109 that is located inside the packaging container main body part bottom section 107 and that has a step structure whose inner circumference side is lower at an upper end. The reference numeral 119 denotes an observation hole provided in the bottom surface of the packaging container main body part 105. A circular elastic member 106 having a hollow part with a diameter larger than the observation hole 119 is installed on the bottom surface of the packaging container main body part bottom section 107, and the sample container 110 is installed thereon. The packaging container main body part bottom section 107 and the packaging container main body part holding section 109 are integrally fixed with a protrusion 117 and an engagement groove 118 extending in the circumferential direction. A flange part 114 of the insertion-type culture container 113 is held on the lower side of the step structure of the packaging container main body part holding section 109. The reference numerals 111 and 112 denote a culture medium and a biological sample such as regenerated tissue, respectively. The first gasket 103 is provided at a position associated with the flange part 114 of the insertion-type culture container 113.

The sample container 110 and the insertion-type culture container 113 are accommodated in the packaging container main body part 105. The packaging container main body part 105 is covered with the packaging container lid part main body 102. Then, the screw ring part 115 is screwed with the screw structure 108 of the packaging container main body part 105 from the outside, so that the packaging container lid part 101 is fixed to the packaging container main body part 105 while being crimped. The reference numeral 116 denotes a middle opening part of the screw ring part 115. The sample container 110 and the insertion-type culture container 113 themselves are open-system culture containers for which the air permeability between the inside and outside of the container is secured. However, the air permeability between the inside and outside of the packaging container 100 is blocked by the packaging container lid part 101.

An example of using a culture dish and an insertion-type culture container that are generally used as the sample containers is shown. Thus, all of the packaging container and the relevant parts thereof are concentrically arranged. For example, a square-shaped sample container may be used unless steric hindrance occurs when being integrally fixed to the packaging container.

It is necessary for the packaging container main body part 105, the packaging container lid part 101, and various elastic members to use materials that can be sterilized by a sterilization process . For example, if polystyrene is used as the material of the packaging container main body part 105 and the packaging container lid part 101, sterilization treatment by gamma-ray radiation or an ethylene oxide gas process is performed before use to realize sterilization. In the above-described example, polystyrene is used. However, it is obvious that any material can be applied as long as it is harmless to the biological sample and can be sterilized. As the various elastic members, silicon, rubber, a foam material, a spongy elastic body, and the like may be used, and it is preferable to use materials for medical use that can be sterilized by gamma-ray radiation or an ethylene oxide gas process and that emit no harmful materials.

The first gasket 103 has elasticity. When the packaging container lid part 101 is crimped to the packaging container main body part 105, the first gasket 103 serves to suppress the movement of the culture medium 111 enclosed into the inside of the packaging container 100 together with the biological sample 112.

The first gasket 103 allows the air to pass through when being not crimped, and desirably suppresses the movement of gas and liquid when being crimped. As the material of the first gasket 103, a spongy elastic body that satisfies the conditions is used such as a foam material. However, any material having the same nature may be used. Further, when the packaging container is opened, the first gasket 103 serves to prevent the culture medium from being leaked outside the packaging container.

The second gasket 104 has elasticity, and suppresses the movement of gas and liquid when being crimped. At the same time, the second gasket 104 prevents bacteria and the like from entering from the outside of the packaging container, and keeps the cleanliness inside the packaging container. Further, in the case of air transportation by aircraft using the packaging container, it is desirable to keep the inner airtightness even under reduced pressure. In the case where the airtightness by the second gasket 104 is insufficient under reduced pressure (reduced to as low as 800hPa in the cabin) at the time of air transportation, an airtight container that keeps the airtightness is further installed outside the packaging container to avoid the effects of the reduced pressure at the time of air transportation. As the material of the second gasket 104, an elastic body that satisfies the conditions is used such as silicon or rubber. However, any material having the same nature may be used.

Further, the first gasket 103 and the second gasket 104 may be attached to the packaging container lid part 102. However, these may be provided to the packaging container main body part 105, or each one may be separately provided to the packaging container lid part 102 and the packaging container main body part 105. This also similarly applies to another packaging container having a different configuration. It should be noted that in the case where the first gasket 103 is provided to the packaging container main body part 105, the flange part 114 is pressed into the packaging container main body part 105 through the circular first gasket 103 when holding the insertion-type culture container 113.

The screw ring part 115 is used to integrally fix the packaging container lid part 101 and the packaging container main body part 105 to each other. The packaging container main body part 105 and the screw ring part 115 are integrally fixed to each other using a screw structure. At the same time, other parts in the packaging container 100 are crimped. It should be noted that as another method of sealing, it is obvious that the container can be held while fixing plural positions by fitting, pins, or spring members.

As the sample container 110 held on the bottom surface of the packaging container main body part 105, for example, a commercially-available culture dish that is generally used for cell culture can be used. There are culture dishes manufactured by Becton, Dickinson and Company, Corning Incorporated, and Greiner-bio-one, and the product to be used is not particularly limited. Further, a temperature-responsive culture dish manufactured by CellSeed Inc can be used. Using a commercially-available culture dish, cellular kinetics such as adhesion, extension, proliferation, and differentiation at the time of cell culture becomes the same. Culture dishes approved as medical equipment for clinical use can be used. Those other than commercially-available culture dishes can be used in accordance with the purpose of the user. There are various types of culture media 111 held in the sample container 110. Examples of supplying nutrients include a KCM culture medium in the case of epidermal cells and corneal epithelial cells, and a 10% serum-containing culture medium in the case of skin fibroblast. Further, examples of supplying no nutrients include a PBS culture medium with the osmotic pressure same as that in cells, normal saline, perfusate used for organ transplantation, and UW solution. The culture medium is appropriately selected in accordance with the conveyance time and conveyance conditions.

FIG. 1C(d) shows the insertion-type culture container 113 having the regenerated tissue 112 after culture. The insertion-type culture container may be a commercially-available cell culture insertion container. There are culture containers manufactured by Becton, Dickinson and Company, Corning Incorporated, and Greiner-bio-one, and the product to be used is not particularly limited. Further, a temperature-responsive cell culture insertion container manufactured by CellSeed Inc can be used. The bottom surface of the cell culture insertion container is of a porous membrane, and has plural holes with a diameter of, for example, about 0.4µm. Accordingly, the culture medium and liquid factors can be moved between the upper layer and the lower layer. It should be noted that the reference numeral 1130 denotes a protrusion extending outward in the radial direction.

The above-described packaging container 100 for conveying a biological sample is used in the case where the manufacturing of the biological sample 112 in a cell processing center is completed and then the packaging container 100 is conveyed to medical institutions and research institutions in each area while accommodating the manufactured biological sample.

The conveyable biological sample packaging container and the biological sample packaging container used for culturing a biological sample in an automatic culture apparatus have packaging container main body parts may have the same structure. In addition, the second packaging container lid part and the first packaging container lid part separately associated with the packaging container main body parts have lid structures having the first gasket 103 and the second gasket 104 may have the same configuration. It should be noted that the packaging container main body part of the packaging container 100 and the packaging container main body part of a packaging container 200 having the same structure may be separately provided as will be described in the following. However, an example of commonly using a single packaging container main body part will be described in the following.

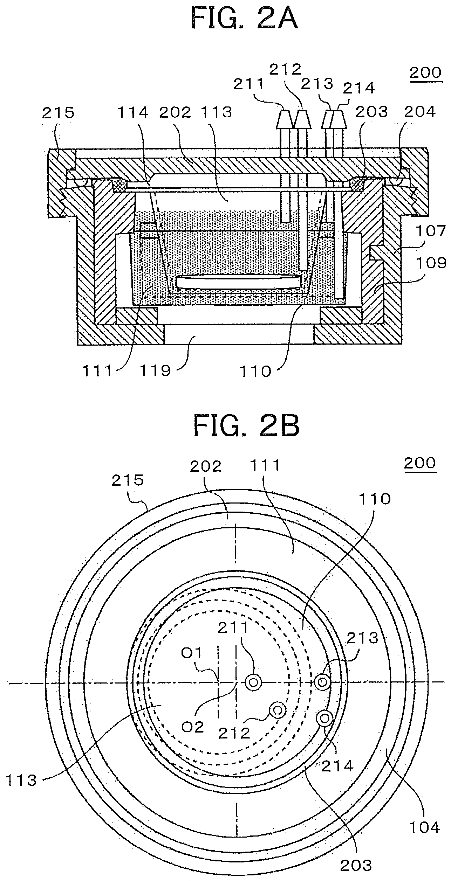

FIG. 2A shows the packaging container 200 for culturing a biological sample, and FIG. 2B shows a plan view thereof. The biological sample packaging container 200 includes the packaging container main body part 105 same as that of the packaging container 100 shown in FIGs. 1A to 1C, a first packaging container lid part 201, and a screw ring part 215. As similar to the packaging container lid part 101, the first packaging container lid part 201 is configured using a transparent first packaging container lid part main body 202, and a first gasket 203 and a second gasket 204 fixed thereto. Further, the packaging container lid part main body 202 is provided with four flow channel tubes (211 to 214).

The first packaging container lid part 201 of the biological sample packaging container 200 in the state of culture is different from the first packaging container lid part in the structure having the plural flow channel tubes. The other configurations thereof are the same as those of the biological sample packaging container 100, and the shapes and sizes of the first gaskets, the second gaskets, and the screw ring parts of the both are the same. Thus, the packaging container lid part of FIG. 2A and the packaging container lid part shown in FIGs. 1A to 1C can be commonly used for the same packaging container main body part 105.

The insertion-type culture container 113 and the culture dish 110 are installed in the packaging container main body part 105 as sample containers. For example, epithelial cells such as oral mucosal cells, corneal epithelial cells, and epidermal cells are sown in the insertion-type culture container 113 serving as the upper layer, and feeder cells such as mouse-derived 3T3 cells are sown in the culture dish 110 serving as the lower layer to culture using a feeder method. During the culture, culture medium replacement is performed so that an old culture medium is discharged and a new culture medium is supplied through the flow channel tubes 211 to 214. The example shows a case in which the supply flow channel tube 211 and the discharge flow channel tube 212 used for the upper layer and the supply flow channel tube 213 and the discharge flow channel tube 214 used for the lower layer, namely, four tubes in total are used. In the example, the culture media are supplied and discharged through different flow channel tubes. Thus, it is possible to avoid a mixture of a new culture medium and an old culture medium.

Further, as shown in FIG. 2B, a center 02 of the culture dish 110 is shifted from a center O1 of the insertion-type culture container 113. This configuration is suitable for performing an automatic culture process by an automatic culture apparatus before a conveyance process. In the automatic culture process, the culture medium is automatically replaced by another. Thus, the flow channel tubes need to be inserted into the culture dish and the insertion-type culture container. The four flow channel tubes are concentrated on one side of the first packaging container lid part 201, and are arranged while being extended in the vertical direction. Thus, the flow channel tubes can be easily inserted.

Next, an operation of installing and removing the first and second packaging container lid parts at/from the packaging container main body part will be described.

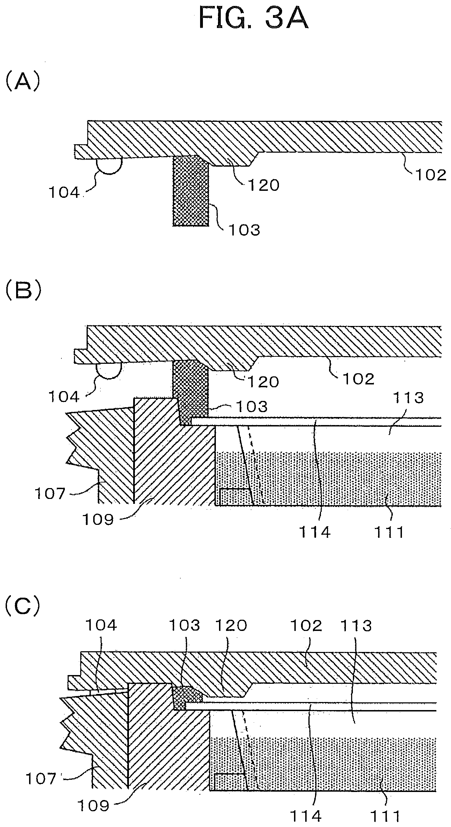

The packaging container lid part is installed at the packaging container main body part immediately before shifting to the conveyance process after the manufacturing of the biological sample in a cell preparation room of a cell processing center is completed. FIG. 3A is a diagram for showing an operation of installing the packaging container lid part at the packaging container main body part 105. FIG. 3A(a) shows the packaging container lid part, and the packaging container lid part main body 102 has a first gasket 103 (203 in FIG. 2A) and a second gasket 104 (204 in FIG. 2A) . Further, the packaging container lid part main body 102 has a circular step part 120 protruding downward on the inner side of the first gasket 103. The first gasket 103 is longer than the second gasket 104. Thus, when the packaging container lid part is installed at the packaging container main body part, the first gasket 103 first abuts on the packaging container main body part (the packaging container main body part holding section 109). On the contrary, when the packaging container lid part is removed from the packaging container main body part, the second gasket 104 is first separated from the packaging container main body part (the packaging container main body part bottom section 107). Specifically, the first gasket 103 is larger than the second gasket 104 in the crushing margin.

FIG. 3A(b) shows a state in which the first gasket 103 first abuts on the packaging container main body part in the operation in which the packaging container lid part is installed at the packaging container main body part. This work is conducted when the culture process is completed as described above. When being not crimped, the first gasket 103 allows the air to flow to the outside from the packaging container main body part, and suppresses the movement of liquid. As long as the culture process is normally conducted, the culture medium does not scatter to the outside of the packaging container, and exists only inside the culture container. Further, the first gasket 103 is long in the vertical direction (sealing direction), and thus the culture medium can be prevented from being moved during the conveyance and at the time of opening the packaging container.

FIG. 3A(c) shows a state in which the second gasket 104 abuts on the packaging container main body part bottom section 107. In this state, the packaging container lid part and the packaging container main body part are integrally fixed to each other using the screw ring part 115. Although the container is conveyed in this state, the second gasket 104 suppresses the movement of gas and liquid during the conveyance, and further prevents bacteria and the like from entering from the outside of the packaging container. Thus, the cleanliness inside the packaging container can be kept. Further, in the case of air transportation by aircraft using the packaging container 100, the inner airtightness is kept even under reduced pressure. In addition, the first gasket 103 is also pressed by the step part 120 of the packaging container lid part main body 102 and the packaging container main body part holding section 109, and is crimped to the flange part 114 of the insertion-type culture container 113 to block the movement of not only gas but also liquid. At the time of conveyance performed in the state of FIG. 3A(c), the culture medium is suppressed from being moved from the inside of the packaging container 100 to an area outside the first gasket 103 and from the outside of the packaging container 100 to an area inside the second gasket 104.

Next, the packaging container lid part is opened to take the biological sample out of the packaging container main body part in an operating room or the like where medical treatment such as transplantation is conducted after the conveyance process is completed.

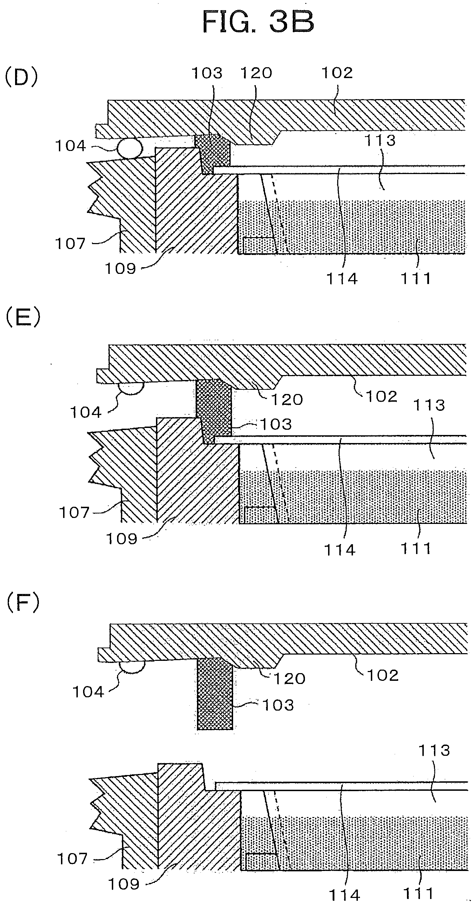

FIGs. 3B are diagrams for showing an operation of removing the packaging container lid part from the packaging container main body part 105. FIG. 3B(d) is a diagram for showing a state immediately before the second gasket 104 does not abut on the packaging container main body part (107) in an operation in which the screw ring part 115 is removed to remove the packaging container lid part from the packaging container main body part after conveyance. The crimped first gasket 103 and second gasket 104 are in a state in which the shapes are being recovered. In the case where the packaging container 100 is inclined during the conveyance and the inner culture medium abuts on the first gasket 103, the culture medium is returned to the original position in accordance with the gravity in many cases. Specifically, the first gasket 103 is long in the vertical direction (sealing direction), and thus the movement of the culture medium can be suppressed during the conveyance and at the time of opening the packaging container. As the first gasket, a spongy elastic body is supposed to be used as described above. The culture medium possibly leaches to an area outside the first gasket and inside the second gasket from air gaps included in the first gasket. In this case, as the crimped first gasket 103 is returned to the original shape in FIG. 3B(d), the leached culture medium can be sucked.

FIG. 3B(e) shows a state in which the second gasket 104 does not abut on the packaging container main body part (107) . At this point, the first gasket 103 still abuts on the packaging container main body part (109). Thus, even if the culture medium is to be moved to the outside of the packaging container due to some cause, the movement is restricted by the first gasket 103, and is not leaked to the outside. The first gasket 103 is shifted to a state in which the first gasket 103 does not abut on the packaging container main body part (109) as shown in FIG. 3B(f) for the first time in a stable state in which the culture medium completely stays in the packaging container, and the packaging container lid part is completely separated from the packaging container main body part.

It should be noted that in the case of a structure in which the first gasket 103 is separated from the packaging container main body part earlier than the second gasket 104 is, when a force to move the culture medium to the outside of the packaging container is applied due to some cause in this state, the culture medium reaches the second gasket. Accordingly, when the packaging container lid part is removed, the inner culture medium is easily leaked to the outside. The outside of the second gasket possibly becomes an unclean state at the time of conveyance, and thus bacteria and the like possibly adhere thereto. If the culture medium is leaked to the outside of the second gasket, there is a risk of biological contamination in which bacteria and the like enter the inside through the leaked culture medium. It means that the biological sample cannot be used for medical treatment. In order to avoid such a situation, the second gasket 104 is separated from the packaging container main body part earlier than the first gasket 103 is in the present invention.

This action prevents the culture medium from being leaked to the outside of the packaging container when the packaging container is opened. As a result, a risk of biological contamination can be suppressed. Further, the state of the biological sample is confirmed using a microscope from the outside of the packaging container if needed at a point before medical treatment after conveyance. The packaging container 100 has transparency, and there is the observation hole 119 provided in the bottom surface of the packaging container main body part 105 on the lower side of the packaging container as shown in FIG. 1A. Thus, the culture dish 110 is exposed. Accordingly, the cells in the packaging container can be observed under the optical conditions same as those at the time of an observation in a manual culture process.

It should be noted that the number of gaskets on the rear surface of the packaging container lid part main body 102 is not necessarily limited to two, but may be one in some cases depending on the shapes of the packaging container main body part 105 and the insertion-type culture container 113 . As similar to the above, the step part 120 of the packaging container lid part main body 102 is not necessarily required. The rear surface of the packaging container lid part main body 102 may be flat according to the use.

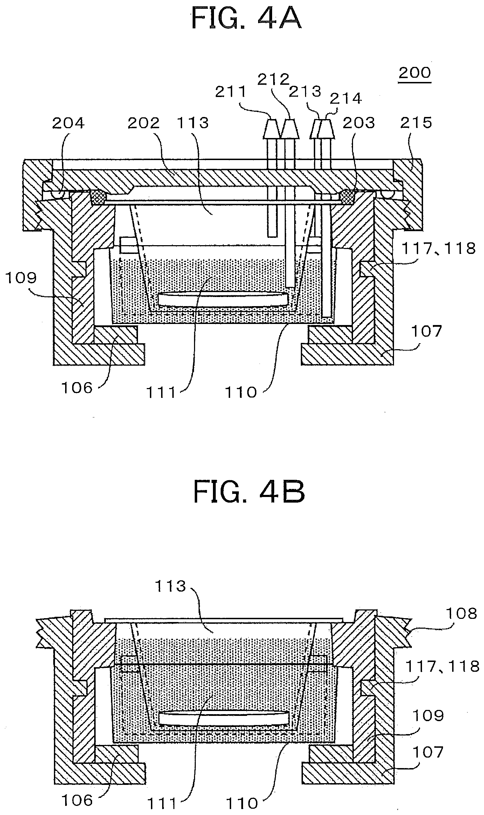

Next, an operation of storing the biological sample in the biological sample packaging container 200 into the packaging container after culture by an automatic culture apparatus or manual culture is performed using the biological sample packaging container 200 will be described using FIGs. 4A to 4D.

First, FIG. 4A shows a state in which culture is performed by the automatic culture apparatus. This state is the same as FIG. 2A, and as the packaging container lid part for the packaging container main body part 105, a lid with the flow channel tubes 211 to 214 provided to the packaging container lid part main body 202 is used for the biological sample packaging container 200. During the culture, an observation by a microscope is regularly performed using the automatic culture apparatus. The culture dish that is a sample container is exposed through the observation hole 119, and thus an observation can be realized under the optical conditions same as those at the time of manual work. During the culture, the flow channel tubes 211 to 214 are connected to closed-system flow channels, and are isolated from the outside of the culture system. Accordingly, closed-system culture can be performed while keeping the cleanliness.

At the stage of shifting to the conveyance process after the culture process is completed, the culture container is aseptically separated from the inside of the automatic culture apparatus to be carried to a safety cabinet. Then, as shown in FIG. 4B, a packaging container lid part main body 202 having the flow channel tubes used for the culture in the biological sample packaging container 200 is removed from the packaging container main body part 105 by operating a screw ring part 215. FIG. 4B shows a state in which the insertion-type culture container 113 having the cultured regenerated tissue 112 and the culture dish 110 are stored in the packaging container main body part 105.

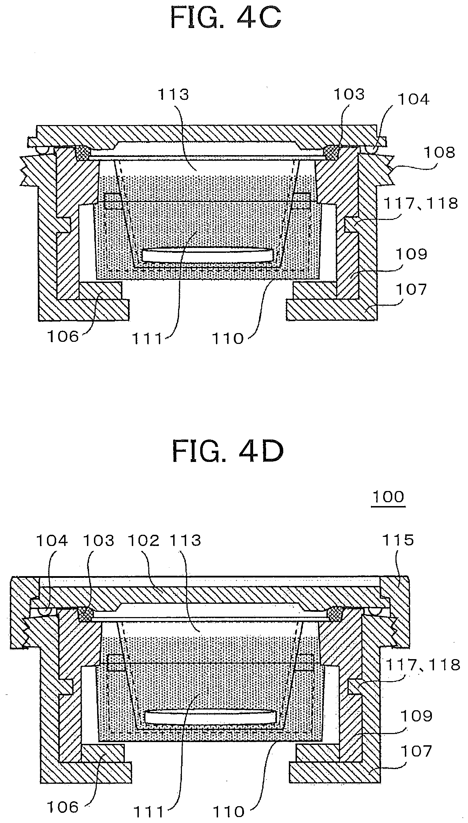

Further, as shown in FIG. 4C, the packaging container main body part 105 is covered with the packaging container lid part main body 102 having no flow channel tubes as the packaging container lid part. Specifically, FIG. 4C shows a state in which the second packaging container lid part 101 is installed at the packaging container main body part 105.

Next, as shown in FIG. 4D, the packaging container lid part is fixed to the packaging container main body part 105 using the screw ring part 115 to form the biological sample packaging container 100. Specifically, FIG. 4D shows a state in which the screw ring part 115 is further installed at the packaging container main body part 105 to form the biological sample packaging container 100 for conveyance with the all parts integrated. In this state, the cleanliness in the packaging container can be kept. Further, in the case of air transportation by aircraft, the inner airtightness is kept even under reduced pressure. Inside the packaging container, the first gasket suppresses the movement of the culture medium to an area between the first gasket and the second gasket. After conveyance, an inspection is conducted in a state in which the airtightness of the packaging container is kept if needed. The bottom surface of the sample container 110 is exposed, and thus an observation by a phase-contrast microscope or the like can be realized under the optical conditions (particularly, focal length) same as those at the time of a normal cell observation in which the sample container 110 is not stored in the packaging container. At this time, the culture medium may be replaced by another for conveyance if needed. An example of the culture medium for conveyance is as shown in FIG. 1A.

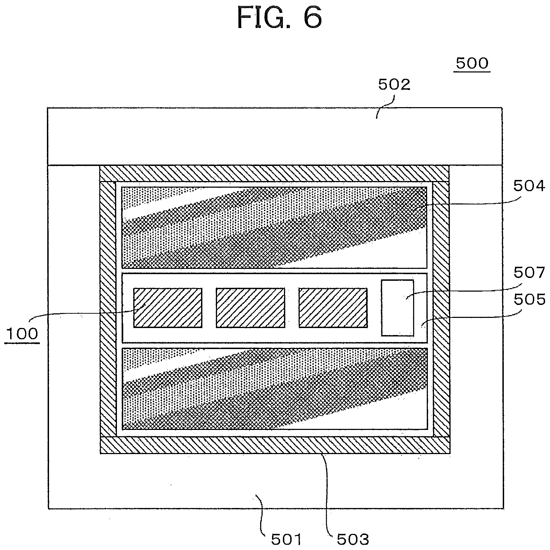

The packaging container lid part is replaced by another, and the container is sealed again as shown in FIG. 4D. Then, the biological sample packaging container 100 is stored in a cell conveyance container to be conveyed. The cell conveyance container will be described later using FIG. 6.

As described above, it is only necessary to replace only the lid part in shifting from the automatic culture process by the automatic culture apparatus to the conveyance process. The packaging container main body part can be used even in the automatic culture process as similar to the conveyance process. The enhancement of the versatility of the packaging container main body part eliminates the transfer work of the biological sample. Thus, the risk of biological contamination and the risk of damaging the biological sample can be reduced.

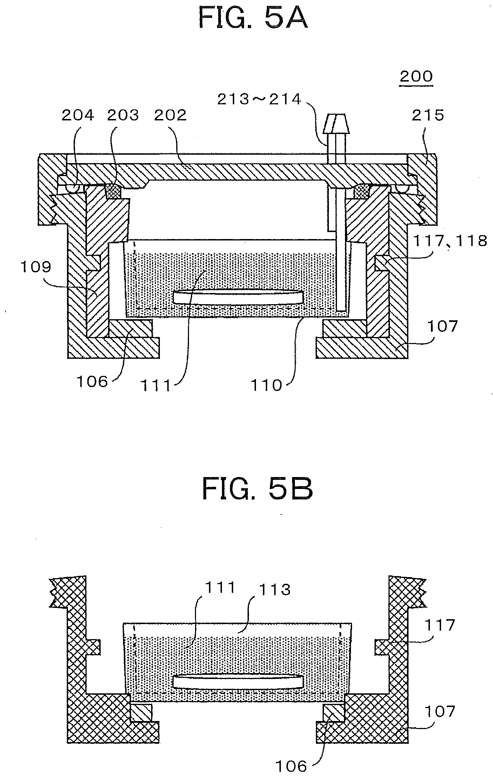

Next, an operation of storing into the packaging container after culture by the automatic culture apparatus or manual culture is performed according to a modified example of the first embodiment will be described using FIG. 5A to FIG. 5C.

FIG. 5A shows a state in which culture is performed by the automatic culture apparatus using the biological sample packaging container 200 as similar to FIG. 4A. However, the insertion-type culture container is not used, but only the culture dish 110 is used as the sample container, which corresponds to one-layer culture. As an example, cardiac muscle cells, fibroblast cells, and the like are sown and cultured. As similar to FIG. 4A, a lid part having the packaging container lid part main body 202 and two flow channel tubes is used as compared to the packaging container lid part shown in FIGs. 1. However, the example shows the one-layer culture, and thus the number of flow channel tubes is two of the supply flow channel tube 213 and the discharge flow channel tube 214 for the sample container that is a culture dish. It should be noted that in the case where it is not necessary to avoid a mixture of a new culture medium and an old culture medium as similar to FIG. 4A, the functions of the supply and discharge flow channel tubes can be provided to one flow channel tube. The observation conditions during the culture are the same as those in FIG. 4A.

At the stage of shifting to the conveyance process after the culture process is completed from the state of FIG. 5A, the culture container is aseptically separated from the inside of the automatic culture apparatus to be conveyed to a safety cabinet. Then, as similar to FIGs. 4B to 4D, the first packaging container lid part having the flow channel tubes used for the culture in the biological sample packaging container 200 is removed from the packaging container main body part 105 by operating the screw ring part 215, and then is replaced by the second packaging container lid part for conveyance to form the biological sample packaging container 100.

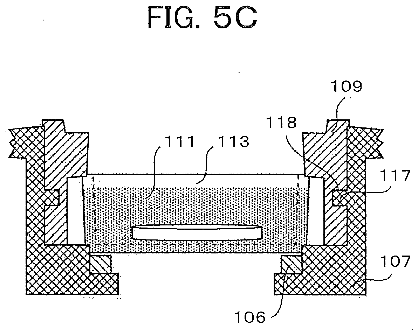

Further, each of FIG. 5B and FIG. 5C shows a procedure in which the culture dish 113 cultured by manual culture is stored into the packaging container to be conveyed. In the case where the insertion-type culture container cultured by manual culture is stored into the packaging container to be conveyed, the packaging container is prepared in advance, and the insertion-type culture container after the manual culture is carried to the inside of the safety cabinet to be aseptically installed into the packaging container main body part. The culture dish 111 having therein the biological sample after completion of the culture and the culture medium 113 is aseptically stored into the packaging container main body part bottom section 107 of the packaging container main body part 105 (FIG. 5B) . The reference numeral 106 denotes an elastic member. Next, the packaging container main body part holding section 109 is aseptically installed as similar to the above (FIG. 5C) . At this time, the packaging container main body part holding section 109 is pressed into the packaging container main body part bottom section 107 while being rotated so that the protrusion 117 of the packaging container main body part bottom section 107 is engaged with the engagement groove 118 of the packaging container main body part holding section 109. Accordingly, the culture dish 111 is fixed to the inside of the packaging container main body part 105. Thereafter, the second packaging container lid part 101 and the screw ring part 115 are attached, and the packaging container is sealed to form the biological sample packaging container 100.

In the case where the feeder cells are needed during the conveyance, the feeder cells are cultured in advance on the culture dish in the packaging container, and the insertion-type culture container after manual culture is installed therein. Then, the culture medium for conveyance is put in, and the second packaging container lid part and the screw ring part are attached to the packaging container main body part as similar to FIGs. 5B and 5C to form the sealed biological sample packaging container to be conveyed.

As described above, the packaging container main body part, the first packaging container lid part obtained by providing the flow channel tubes to the packaging container lid part, and the second packaging container lid part having no flow channel tubes that is compatible with the lid are provided, and one-layer culture and two-layer culture are performed by the automatic culture apparatus using the first packaging container lid part. At the time of conveyance, only the replacement by the second packaging container lid part having no flow channel tubes enables culture and conveyance of the biological sample while keeping the cleanliness.

FIG. 6 shows a configuration example in the case where the sample container stored into the packaging container 100 is conveyed using a cell conveyance container 500 the inner temperature of which can be kept constant by a heat storage material. The cell conveyance container 500 is configured using a cell conveyance container main body 501 in which inner constitutional parts are stored and a cell conveyance container lid 502, and a heat insulation material 503 is disposed on the inner side thereof. Further, heat storage material boxes 504 with a heat storage material enclosed are disposed on the inner side of the heat insulation material 503. The sample container stored in the packaging container 100 and a monitoring device 507 that measures the temperature, pressure, and oscillation during the conveyance which are disposed in a sample container storage part 505 are disposed at the position sandwiched between the storage material boxes 504.

As the heat storage material, it is preferable to use a pure substance having a constant melting point, or a substance with a large heat capacity and a small temperature change in the melting point (for example, ±1°C or smaller). Accordingly, the variation range of the inner temperature during the conveyance can be reduced, and the impact of the temperature on the biological sample is reduced. As an example of the heat storage material, there is hydrocarbon that is a pure substance. For example, the melting point of hydrocarbon with a chemical formula of C₂₀H₄₂ is 36. 4°C. Hydrocarbon having a different number of C differs in the melting point. Accordingly, the value of the temperature in the cell conveyance container that is kept constant can be changed by selecting the type of hydrocarbon. The environments at the time of conveyance can be confirmed after the conveyance by the monitoring device 507.

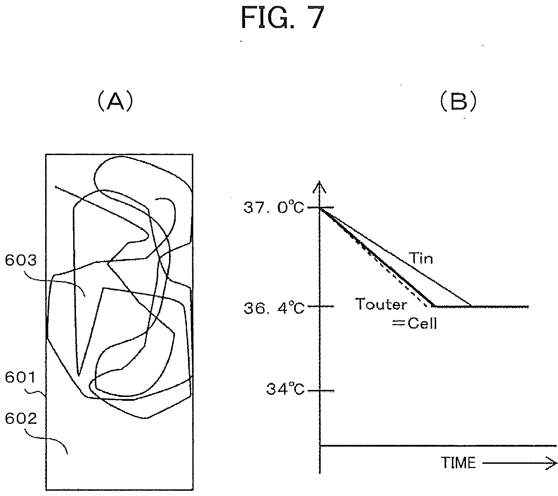

FIGs. 7 are diagrams each showing a mechanism of equalizing the heat distribution of the heat storage material stored in the heat storage material box 504 shown in FIG. 6. In FIG. 7(a), a heat storage material 602 is enclosed in a heat storage material box 601. A linear metal 603 is installed into the heat storage material box 601. The linear metal 603 is sufficiently small in volume and weight, and does not increase the entire weight of the cell conveyance container at the time of conveyance. Further, the linear metal 603 is small in volume, and does not largely decrease the load amount of the heat storage material. Metal such as aluminum having high thermal conductivity is used.

FIG. 7 (b) shows a relation among a temperature Tₒᵤₜₑᵣ of the exterior surface of the heat storage material box 601, an internal temperature Tᵢₙ of the heat storage material 602, a temperature Cell of the packaging container 100 in the heat storage material box 601, and time. The heat storage material 602 is affected by changes in the outside temperature Tₒᵤₜₑᵣ, and changes (reduces in general) the temperature Tₒᵤₜₑᵣ from the outside of the heat storage material 602. Therefore, the time when the inside of the heat storage material 602 changes the temperature Tᵢₙ is later than the time when the outside of the heat storage material 602 changes the temperature. This means non-uniformity of the temperature in the heat storage material box 601. The heat of the heat storage material can be efficiently emitted by the installation of the linear metal 603. As a result, a period of time in which the temperature Cell of the entire cell conveyance container 500 is kept high can be expected to be longer.

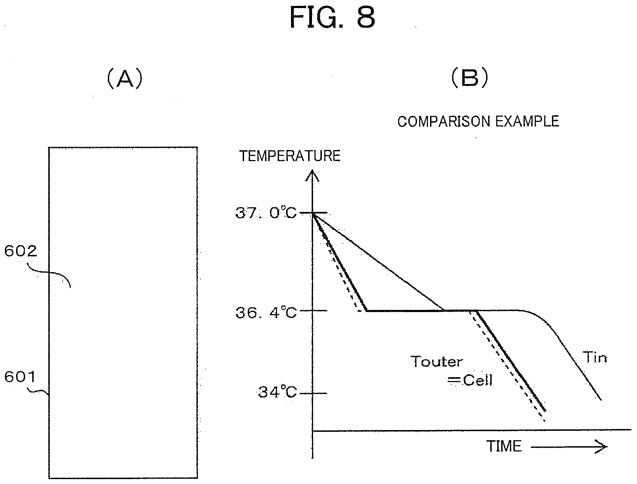

FIG. 8 (a) shows a state in which only the heat storage material 602 is enclosed into the heat storage material box 601 as a comparison example. The heat of the heat storage material cannot be efficiently emitted, and a period of time in which the temperature Cell of the entire cell conveyance container 500 is kept high becomes shorter as shown in FIG. 8(b).

It should be noted that in addition to the configuration of FIG. 7(a), a stirrer may be installed into the heat storage material box 601. The stirrer is, for example, a spherical object. When the bottom surface of the heat storage material box 601 is not horizontal, namely, when the cell conveyance container is being conveyed, the position relative to the heat storage material box 601 can be easily changed. The movement of the stirrer in the heat storage material 602 generates a stirring flow to the heat storage material 602, and the heat distribution of the heat storage material 602 is equalized. Based on the above, the heat distribution of the heat storage material 602 is further equalized by a combination of the linear metal 603 and the stirrer.

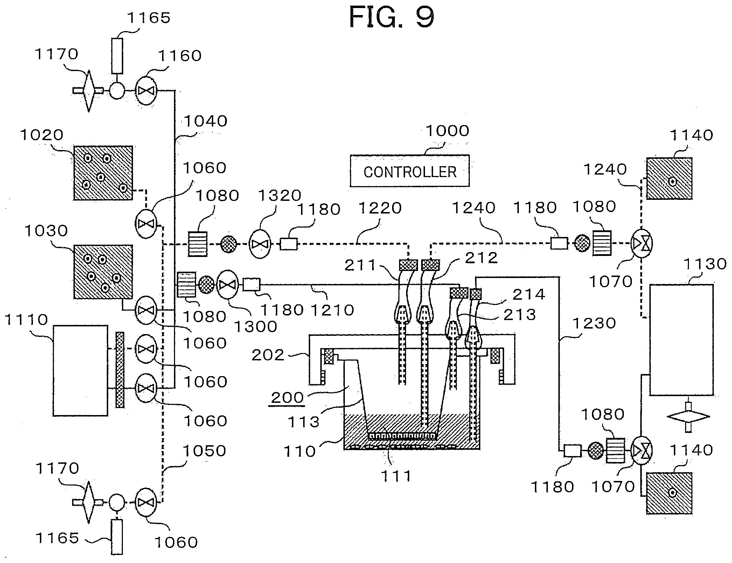

Next, a configuration example of the automatic culture apparatus will be described with reference to FIG. 9. FIG. 9 is a diagram for explaining entire flow channels when the closed-system biological sample packaging container 200 is cultured. The biological sample packaging container 200 is provided with four flow channel tubes, namely, the supply flow channel tube 211 used for the upper layer of the biological sample packaging container 200, the discharge flow channel tube 212 of the upper layer, the supply flow channel tube 213 used for the lower layer, and the discharge flow channel tube 214 of the lower layer. In the automatic culture apparatus, a cell bag 1030 is connected through an introduction part 1040 of a second flow channel circuit (flow channel tube) between one or more closed-system biological sample packaging containers 200 and the supply side of each biological sample packaging container 200, and another cell bag 1020 is connected therebetween through an introduction part 1050 of a first flow channel circuit (flow channel tube). Further, a culture medium bag 1110, gas supply parts 1165, and filters 1170 are connected to these biological sample packaging containers 200 through the introduction parts of the first and second flow channel circuits (flow channel tubes), plural two-way valves 1060, fluid movement control mechanism parts 1080, and multiple branching parts. Each of the fluid movement control mechanism parts 1080 functions as a pump to move a fluid. Further, on the supply side of each culture container 200, provided are a guiding branch 1210 of the second flow channel circuit, a guiding branch 1220 of the first flow channel circuit, a first electromagnetic valve 1300, a second electromagnetic valve 1320, and aseptic desorption parts 1180. To the outlet side of each culture container 200, a guiding branch 1230 of the second flow channel circuit and a guiding branch 1240 of the first flow channel circuit are connected through the aseptic desorption parts 1180, and these flow channel circuits are further connected to a drainage bag 1130, drainage collection bags 1140, and a filter through the fluid movement control mechanism parts 1080 and three-way valves 1070.

In the automatic culture apparatus provided with such flow channel circuits, each two-way valve 1060, each fluid movement control mechanism part 1080, each first electromagnetic valve 1300, each second electromagnetic valve 1320, and each three-way valve 1070 are controlled by a controller 1000 according to a preliminarily-given control protocol on the basis of a predetermined sequence. Accordingly, the first and second flow channel circuits are controlled so that culture media always flow to the culture container 200 through the four flow channel tubes 211 to 214 in one direction, and a new culture medium is supplied after an old culture medium is discharged.

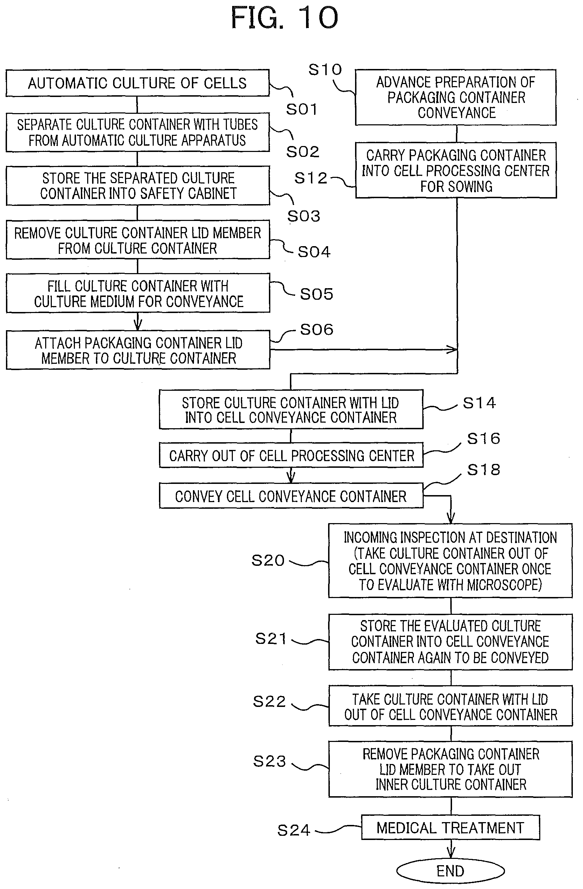

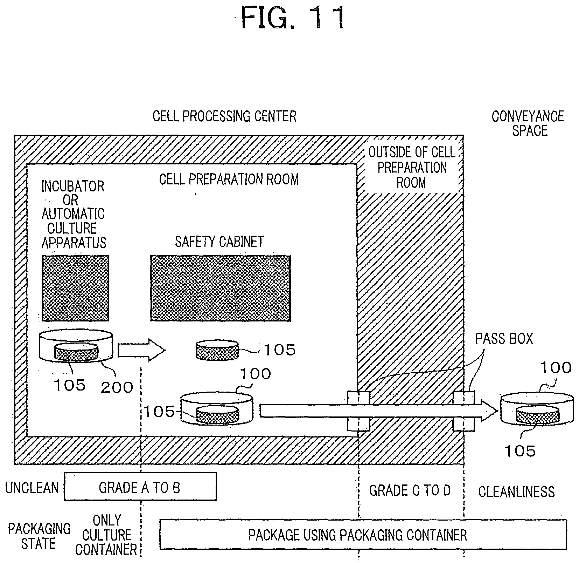

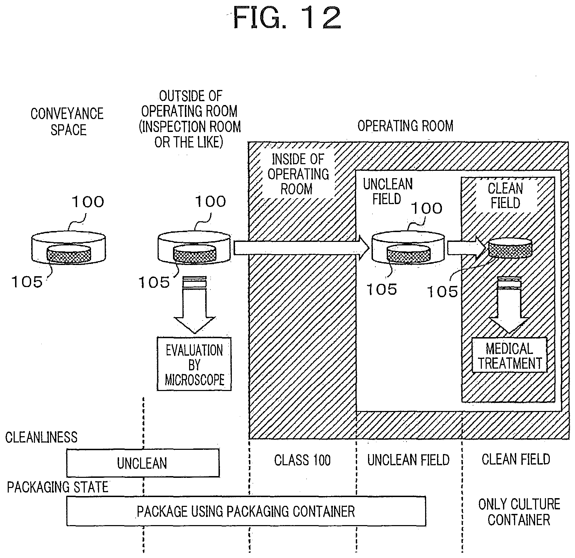

Next, using FIG. 10 to FIG. 12, a series of procedures in which the culture container cultured in the automatic culture apparatus is used as the packaging container shown in the embodiment and the cells are conveyed from a cell processing center such as a CPC to an operating room will be described. FIG. 10 is a diagram for showing an example of procedures in which the culture container is used as the packaging container to be conveyed from a CPC. FIG. 11 shows a process in which the sample container is carried out of the cell processing center, and FIG. 12 shows a process in which the sample container is carried into the operating room. Hereinafter, the process will be described in accordance with the procedures of FIG. 10.

### <Step S01: culture of cell>

The culture of cells is performed in the automatic culture apparatus shown in FIG. 9 using the biological sample packaging container 200. The packaging container main body part 105, the first packaging container lid part 201 with proper flow channel tubes, and the proper sample containers 110 and 112 are aseptically assembled to form the biological sample packaging container 200 which is installed in the automatic culture apparatus to be used for culture. It should be noted that all parts may be assembled at a factory where the parts are manufactured and may be carried into the cell processing center in a completely-sterilized state to be aseptically installed in the automatic culture apparatus. Further, as the proper sample containers, for example, an insertion-type culture container and a culture dish are used in the case of two-layer culture, and, for example, a culture dish is used in the case of one-layer culture. As the lid part with proper flow channel tubes, the number and arrangement of flow channel tubes are selected in accordance with, for example, the conditions of the two-layer culture or one-layer culture, and further in accordance with whether or not a mixture of an old culture medium and a new culture medium is avoided when replacing the culture medium. The cells are manually cultured in some cases.

As shown in FIG. 11, the cells are cultured in a cell preparation room of the cell processing center such as a CPC using the culture container (biological sample packaging container) 200. The cleanliness of the room is, for example, Grade B according to the EU-GMP standard. In the case of manual culture, the sample containers 110 and 113 at the time of culture are put in an incubator while being held in the packaging container main body part 105, and are taken out if needed. Accordingly, the culture medium is replaced by another in a safety cabinet with a cleanliness of Grade A. Further, the configuration and proliferation of the cells in the biological sample packaging container 200 are regularly and non-invasively evaluated by a microscope. In the case of culture using the automatic culture apparatus, the sample containers are installed in the cell preparation room of, for example, Grade B, and are cultured in a state where the cleanliness similar to that at the time of the manual culture is kept. The replacement of the culture medium and a cell observation are automatically performed if needed.

Equipment such as a heat block whose temperature is set at the value same as that of a constant-temperature bath is preliminarily prepared in the safety cabinet if needed. Using the equipment, the temperature of the culture container is kept constant until the packaging container main body part 105 of the culture container is completely stored into the cell conveyance container 500.

### <Step S02 to S03: storage of culture container into safety cabinet>

The culture container (biological sample packaging container) 200 that was automatically cultured in the automatic culture apparatus is moved to the safety cabinet. Alternatively, the culture container (biological sample packaging container) 200 that was manually cultured in the constant-temperature bath is moved to the safety cabinet. It should be noted that when the packaging container main body part 105 of the culture container is removed from the automatic culture apparatus, the flow channel tubes of the lid member are aseptically cut off.

### <Step S04 to S06: replacement of packaging container lid>

The packaging container main body part 105 storing, in the sample containers 110 and 113, the sample conveyed to the operating room after completion of the manual culture or the culture by the automatic culture apparatus is packaged in the safety cabinet as shown in FIG. 11 to form the biological sample packaging container 100. Specifically, in the case of culture using the automatic culture apparatus, the first packaging container lid part 201 is removed from the packaging container main body part 105 of the biological sample packaging container 200 moved to the safety cabinet, and the culture container is filled with the culture medium for conveyance. Next, the second packaging container lid part 101 is attached to the packaging container main body part 105 to form the biological sample packaging container 100.

The packaging procedure is as shown in FIGs. 4A to 4D and FIGs. 5A to 5C. The packaging container 100 is preliminarily sterilized, and thus is in a sterilized state. Accordingly, the inside of the sample container and the inside and outside of the packaging container have the same level of cleanliness as Grade A when the packaging is completed.

In the case of manual culture and in the case where the insertion-type culture container is to be conveyed, the insertion-type culture container is aseptically put in the packaging container main body part 105 of the packaging container 100 to be filled with the culture medium for conveyance . Next, the packaging container lid part is attached to form the packaged biological sample packaging container 100.

The all operations are aseptically and quickly performed. Further, it is necessary to be careful of avoiding a leakage of the culture medium. After the operations or during the waiting time, the heat block or the like is used in order to minimize a decrease in the temperature of the culture container.

It should be noted that the culture medium used at the time of the culture in the safety cabinet is replaced by another for conveyance if needed. Further, in the case of culture using the automatic culture apparatus, particularly, in the case where the automatic culture apparatus performs culture using closed-system flow channels, the flow channel tubes installed at the lid of the culture container are aseptically separated first to be switched to the packaging container having no flow channel tubes. As one example, in the case where the flow channel tubes are installed at only the lid part in the closed-system culture container of the automatic culture apparatus, only the lid is switched to one for conveyance in the safety cabinet to enable the conveyance of the container.

### <Step S10: advance preparation of conveyance>

In parallel with Steps S01 to S06, advance preparation for conveyance of cells is carried out. In the case where the insertion-type culture container or culture dish cultured using the automatic culture apparatus is to be conveyed, the lid part is sterilized together with the packaging container 100 of the cell conveyance container 500. The packaging container 100 is preliminarily packaged in an autoclave bag. In this state, the packaging container 100 is sterilized by a sterilization process. As the method of the sterilization process, an autoclave process, an ethylene oxide gas process, a gamma-ray radiation process, or the like is used, and the method that does not change the nature of the packaging container by performing the sterilization process is selected. For example, if the material is polystyrene, the gamma-ray radiation process is employed.

The heat storage material box 504 with the heat storage material enclosed is put in the incubator to accumulate heat in the heat storage material, and is placed in a static manner until the temperature is stabilized. As an example, in the case of hydrocarbon C₂₀H₄₂ whose melting point is 36.4 °C, and further if the outside temperature in the conveyance is mostly 36.4 °C or lower, the temperature of the incubator is set at 37 °C. Because the outside temperature is lower than the melting point of C₂₀H₄₂ during the conveyance, and heat is discharged from the inside of the cell conveyance container 500 to the outside. On the contrary, in the case where the outside temperature in the conveyance is mostly 36.4 °C or higher, the temperature of the incubator is set at 36 °C. Because the outside temperature is higher than the melting point of C₂₀H₄₂ during the conveyance, and heat enters the inside of the cell conveyance container 500 from the outside.

### <Step S12: carrying into and sowing in cell processing center>

Next, the sterilized packaging container 100 is carried into the predetermined cell preparation room in the cell processing center.

When moving the container between the rooms in the cell processing center, it is necessary to allow the container to pass through pass boxes in order to keep the cleanliness of the rooms and to prevent cross-contamination as shown in FIG. 11. When allowing the container to pass through the pass boxes, ethanol is sprayed for disinfection from the outside of a sterilized bag used at the time of sterilization, and the container is put in the pass boxes. Then, the container is taken out from the door on the side of the room to which the container is moved. After arriving at a cell culture area, ethanol is sprayed around the sterilized bag for disinfection, and the container is put in the safety cabinet. Thereafter, the packaging container is aseptically taken out so as not to be contacted with the outside of the package.

In the case where the insertion-type culture container that was manually cultured is to be conveyed together with the feeder cells, the feeder cells are preliminarily sown in the culture dish that is the sample container held in the packaging container main body part 105 in the packaging container by using the sterilized packaging container 100. It is desirable to culture at least until the feeder cells adhere. In this case, the feeder cells are sown, and then culture is aseptically performed in the incubator for a few hours. In the case where the insertion-type culture container is not conveyed together with the feeder cells, the flow proceeds to Step S14. Even in the case where the manually-cultured culture dish is conveyed, the flow proceeds to Step S14.

### <Step S14: storage into cell conveyance container>

Next, the culture container 100 packaged in Step S06 is stored into the culture container storage part 505 of the preliminarily-prepared cell conveyance container 500. Next, the heat storage material box 504, the culture container storage part in which the packaged culture container 100 is stored, and the monitoring device 507 are stored into the cell conveyance container 500. The monitoring device 507 is turned on before storage to start the measurement, and the temperature, pressure and impact are measured throughout the all processes during the conveyance.

### <Step S16: carrying to the outside of cell processing center>

The cell conveyance container 500 is carried from the room where the cells are cultured to the outside of the cell processing center. When moving the container between the rooms, ethanol is sprayed for disinfection, and the container is allowed to pass through the pass boxes to prevent cross-contamination.

The packaging container main body part 105 is stored into the packaging container 100, and then passes through the pass boxes in this state. Finally, the packaged sample container (biological sample packaging container 100) is carried to the outside of the cell processing center. In this case, the temperature and pressure are kept constant if needed, and the packaged sample container is stored into the cell conveyance container that suppresses oscillation and impact. It should be noted that the cleanliness in the conveyance environments outside the cell processing center is not controlled, and thus there is a possibility that organisms or particles such as bacteria adhere to the outside of the packaging container located on the outermost side. On the other hand, the sample container and the like in the packaging container are in an unopened state during the conveyance, and thus the cleanliness of Grade A can be kept.

### <Step S18: conveyance of cell conveyance container>

In accordance with the position of the operating room as the destination, the cell conveyance container 500 is conveyed by selecting means of transportation. As the means of transportation, there are mainly vehicles, railroads, aircrafts, and conveyance in hand. During the conveyance by vehicles, railroads, and aircrafts, it is desirable to fix the cell conveyance container to the floor if needed to prevent the cell conveyance container from being overturned. Further, in the case of conveyance in hand, workers of conveyance take care of minimizing the oscillation of the cell conveyance container 500.

The culture container (biological sample packaging container 100) is being filled with the culture medium. As possible impacts on the cells occurring when the culture container is inclined during the conveyance, the cells enter a gas phase and dry, the cells are affected by surface tension generated between a gas phase and a liquid phase, and convection occurs in a liquid phase to cause shear stress in the cells. As the gas phase part in the culture container is smaller, the impacts can be reduced. In the conveyance method the culture container is being filled with the culture medium as much as possible. Thus, it is conceivable that the gas phase does not largely affect during the conveyance. Further, the direction of gravity applied to the biological sample is changed as compared to that at the time of culture in the cell processing center due to the inclination during the conveyance. In terms of the impact, as long as the cell conveyance container stands upright, the direction of gravity is the same as that at the time of culture in the cell processing center. Further, a period of time the cell conveyance container does not stand upright is not too long. Accordingly, it is conceivable that the impact by gravity is small.

### <Step S20: incoming inspection at destination>

An incoming inspection for the biological sample is conducted at the destination. After the cell conveyance container 500 arrives at the operating room that is the destination, the temperature around the biological sample during the conveyance is checked first. Data of the monitoring device is moved to a PC or the like to be evaluated. Next, the state of the conveyed biological sample is checked. There are various inspection methods. However, a sample used for medical treatment needs to be non-invasively inspected. Specifically, the inspection needs to be conducted by a method in which the biological sample is not contacted directly or through the culture medium. In the case of using the packaging container 100 of the present invention, cell configurations, cell adhesion, and the like are evaluated by an inverted phase-contrast microscope, and the thickness of the biological sample is measured as the non-invasive inspection method. All the samples can be evaluated. Further, the culture container is once taken out of the cell conveyance container at the time of the inspection, the sample is quickly evaluated by a microscope, and the culture container is immediately stored into the cell conveyance container again. Accordingly, the culture container is kept at the same temperature after the inspection. It should be noted that a sample that is not used for medical treatment may be checked in detail by an invasive inspection. In this case, various processes are performed for the biological sample, and the number of cells, the cell survival rate, the tissue structure, and the manifestation condition of particular protein can be checked.

FIG. 12 is a diagram for showing a procedure in which the packaging container 100 is carried into the operating room. After the conveyance, the state of the biological sample is evaluated first. The sample is not possibly evaluated in the case where the operating room is located on the same property and in the case where surgery is performed right after the biological sample is manufactured. In the case where the sample is evaluated, it is evaluated on the basis of the result whether or not the sample can be used for medical treatment. In this case, an non-invasive evaluation method needs to be performed. Because the quality of the biological sample is changed in an invasive evaluation method. Further, it is desirable to conduct a total inspection. The reason is as follows: The same cell source is used for the cultured biological samples, the cultured biological samples are manufactured through the same manufacturing process, and thus the quality thereof is the same even after the culture. However, the quality of the cells can be easily changed due to a slight change in environments. Accordingly, an observation by a microscope that is the above-described non-invasive evaluation method is performed in the embodiment while the sample container is being packaged.

### <Step S21 to S24: medical treatment>

As a result of the evaluation, if it can be confirmed that the conveyed sample is suitable for medical treatment, the preparation of the medical treatment is started. It is likely to take about one day to prepare the medical treatment. In addition, all medical institutions do not necessarily have equipment such as a constant-temperature bath. In that case, the culture container is kept in the cell conveyance container to keep the temperature and cleanliness until the medical treatment is started even after arriving at the medical institutions.

If the preparation of the medical treatment is completed, the cell conveyance container is moved to a room (hereinafter, referred to as an operating room) where the medical treatment is conducted. If the cell conveyance container arrives at the operating room, the culture container packaged in the packaging container 100 is taken out. The culture container is put in the constant-temperature bath installed in the operating room in this state if needed, and is kept under predetermined temperature. For example, in the case where the culture container has a temperature-responsive culture surface, cryogenic treatment (as an example, exposed under 20°C for 30 minutes) is performed before the medical treatment is conducted, and the biological sample adhering to the temperature-responsive culture surface is peeled off.

Next, the biological sample is taken out of the culture container. The exterior of the packaging container 100 has passed through everyday space, and thus there is a high possibility that organisms or particles such as bacteria adhere thereto. Accordingly, the packaging container 100 is opened stepwise so as to keep the inside of the packaging container clean. First, a worker in an unclean field in the operating room wipes the exterior of the packaging container 100 with a clean cloth using a disinfectant such as ethanol or popidone iodine. Next, the packaging container lid part is removed. Then, a worker in a clean field aseptically takes out the biological sample with tweezers or the like. In this case, the biological sample is aseptically taken out so as not to be contacted with the culture container. In the case where the biological sample is put in the insertion-type culture container, the biological sample is not directly taken out of the packaging container 100, but the insertion-type culture container can be taken out first. Thereafter, the biological sample is taken out. It should be noted that the embodiment is not limited to the above, but a multiple packaging configuration can be adapted to the exterior of the packaging container 100 in order to carry the container into facilities with segmentalized cleanliness levels.

The procedure of the medical treatment will be further explained using FIG. 12. If it is determined that the sample can be used for the medical treatment, the preparation of the medical treatment is started. The preparation for a patient who receives the medical treatment is started. Thereafter, the sample container is carried to the operating room. The operating room has the cleanliness of Class 100 according to, for example, the Federal Standard. The operating room has a clean field and an unclean field. The exterior of the sample container carried into the operating room is first disinfected using ethanol or the like. Then, the packaging container 100 is opened in the unclean field. In this case, it is necessary to take care not to contact the exterior of the packaging container and the clean sample with each other. Further, it is necessary for the inner culture medium not to be leaked. Then, a worker who handles only equipment in the clean filed takes out only the sample container. Finally, the biological sample is taken out of the sample container in the clean field. The biological sample is used for the medical treatment. It should be noted that the biological sample may be aseptically and directly taken out of the inside of the packaging container 100 right after the packaging container 100 is opened.

The packaging container includes the packaging container lid part having the first gasket to suppress the movement of the culture medium and the second gasket to suppress the movement of gas and liquid and to keep the cleanliness in the packaging container. Thus, it is possible to avoid a leakage of the inner culture medium when being conveyed to the operating room or being opened. As a result, the cleanliness of the biological sample can be kept.

The packaging container 100 can be used for a sample container for which the similar cleanliness is required and into which precision parts such as semiconductor chips are introduced.

It is possible to provide a biological sample packaging container that realizes the sealing performance and cleanliness as a packaging container and that can avoid a leakage of the culture medium at the time of an non-invasive inspection and being opened, and a conveyance method thereof.

Further, the packaging container lid part of the packaging container main body part is replaced by a lid member having flow channel tubes that enable the movement of liquid and gas to/from the outside. Accordingly, culture can be performed using an automatic culture apparatus that can perform all or any one of culture medium replacement in an airtight state, a microscope observation, and automatic culture.

Further, after the culture by the automatic culture apparatus, the lid member attached at the time of the automatic culture is replaced by the packaging container lid part to be packaged. In this state, the container can be conveyed while keeping the cleanliness of the inner sterilized substance.

There are various possible containers other than the biological sample packaging container 100 described above. These biological sample packaging containers will be described below.

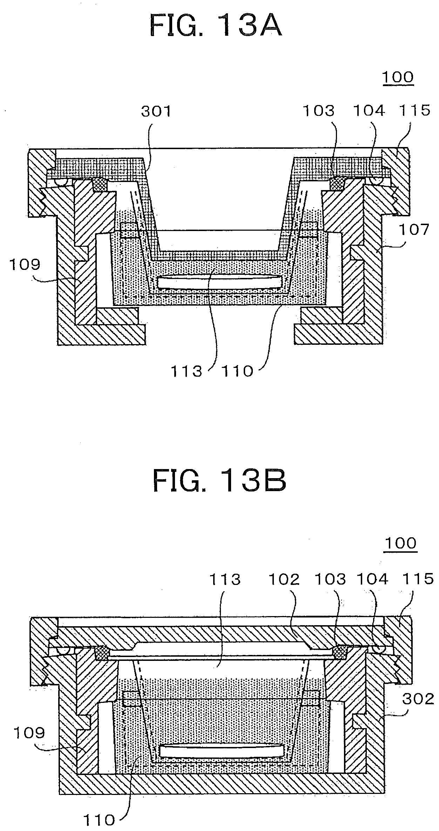

A biological sample packaging container 100 shown in FIG. 13A has a dent part 301 at a packaging container lid part 102. The dent part 301 protrudes towards a sample container 110. The outer diameter of the dent part 301 is smaller than the inner diameter of the sample container 110, so that when packaging container main body parts (107 and 109) and the packaging container lid part 102 are integrated, steric hindrance does not occur to the sample container. As an example, the bottom surface of the dent part 301 has a plate-like flat structure. When the sample container is sealed with the packaging container main body part and the packaging container lid part, the dent part 301 is brought into contact with a culture medium 113 of the sample container 110. Accordingly, a space where the culture medium 113 can be moved due to the oscillation, impact, and inclination of the sample container 110 during the conveyance becomes smaller. Accordingly, a stirring flow generated in the culture medium during the conveyance is reduced. As a result, shear stress that is possibly generated to the biological sample due to the stirring flow is reduced, and the possibility of damaging the biological sample can be suppressed.

It should be noted that in the case where such a packaging container lid part has the dent part to suppress the shear stress from being generated to the biological sample, it is desirable that the air can pass through when being not crimped. The first gasket 103 provided at the packaging container lid part 102 allows the air to pass through when being not crimped as described above, and it is desirable to suppress the movement of gas and liquid when being crimped. As the material of the first gasket 103, a spongy elastic body that satisfies the conditions is used such as a foam material. However, any material having the same nature may be used. Further, when the packaging container is opened, the first gasket 103 serves to prevent the culture medium from being leaked outside the packaging container.

Further, as the biological sample packaging container for the automatic culture apparatus adapted to the biological sample packaging container 100 shown in FIG. 13A, for example, the biological sample packaging container 200 shown in FIG. 2A can be used.

Next, FIG. 13B shows the biological sample packaging container 100 configured in such a manner that the bottom surface of a packaging container main body part bottom section 302 has no opening so as not to expose the sample container. As shown in FIG. 1A, in the case where the bottom surface of the sample container 110 is exposed from the packaging container main body part bottom section 107, the cells can be observed by an ordinary phase-contrast microscope under the optical conditions same as those at the time of a normal cell observation in which the sample container is not stored in the packaging container. In the example, the sample container is not exposed from the packaging container main body part bottom section 302, and thus the optical conditions at the time of the observation differ. In particular, a distance by which an objective lens can be moved closer to the sample container differs from FIG. 1A due to the packaging container main body part bottom section, and thus the focal length is changed. Further, the transparency is reduced due to the packaging container main body part bottom section. Although the optical conditions are changed, it is not necessary, as advantages, to provide the elastic member that is supposed to be necessary when the sample container is fixed to the packaging container main body part bottom section in FIG. 1A. The number of parts can be advantageously reduced. It should be noted that the biological sample packaging container 200 for the automatic culture apparatus adapted to the biological sample packaging container 100 is configured in such a manner that the bottom surface of the packaging container main body part bottom section has no opening as similar to the biological sample packaging container 100.

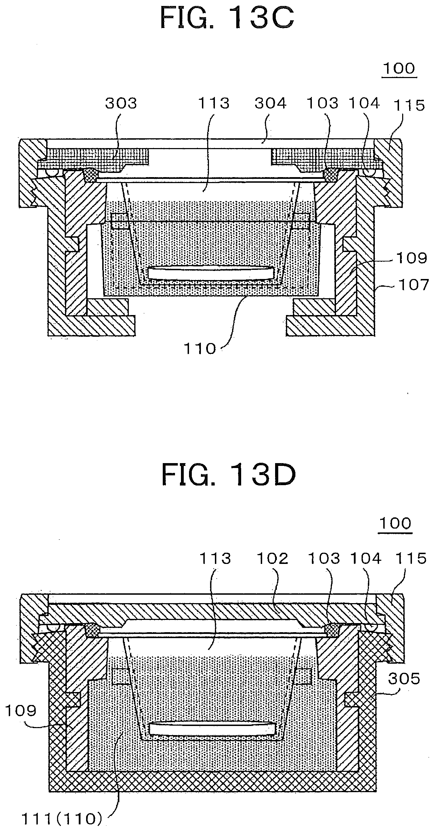

Further, FIG. 13C shows an example of the biological sample packaging container 100 in which a part of a packaging container lid part 303 is formed using a gas permeable membrane 304. Accordingly, gas such as oxygen can be taken in from the outside of the packaging container 100 during the conveyance. The biological sample is configured using various cells, and for example, cardiac muscle cells are high in oxygen requirement. In the case where such cells are to be conveyed, oxygen is supplied from the outside of the packaging container through the gas permeable membrane, so that the state of the biological sample after the conveyance is improved. Further, pH of the culture medium can be controlled by supplying carbon dioxide from the outside. As described above, the gas permeable membrane is employed to the packaging container lid part, so that it is possible to provide the packaging container that conveys the biological sample such as cells that are high in oxygen requirement while keeping a good condition. It should be noted that as the biological sample packaging container 200 for the automatic culture apparatus associated with the biological sample packaging container 100 of FIG. 13C, for example, the biological sample packaging container 200 shown in FIG. 2A may be used. Alternatively, a part of the packaging container lid part may be formed using the gas permeable membrane. Then, the air containing an adequate amount of oxygen or carbon dioxide is supplied from the outside of the biological sample packaging container 200.

Further, FIG. 13D shows a case in which the sample container 110 is not used for the biological sample packaging container 100, but only a packaging container main body part bottom section 305 is used. The sample container for which the cellular kinetics is already known can be used in FIG. 1A. However, in the case where the packaging container main body part bottom section 305 is used, the already-known sample container and the cellular kinetics do not always match. Further, since the sample container is exposed in FIG. 1A, the observation conditions by a microscope can be conformed to those in the case where the already-known sample container is used. However, the observation conditions do not match in the example. Instead, as advantages, it is not necessary to provide the elastic member and the sample container that are supposed to be necessary when the sample container is fixed to the packaging container main body part bottom section in FIG. 1A. The number of parts can be advantageously reduced.

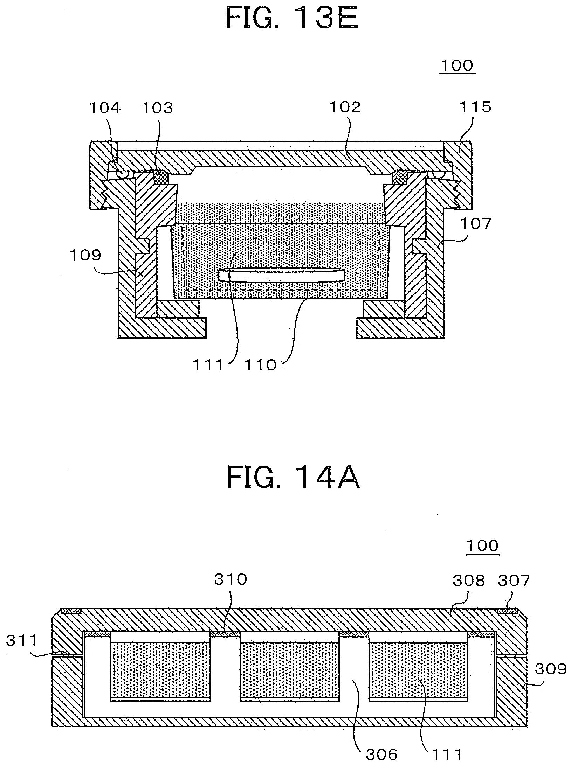

Next, FIG. 13E shows a case in which a biological sample 112 is put not in an insertion-type culture container but a sample container that is a culture dish 110 as compared to the case of the biological sample packaging container 100 of FIG. 1A. As shown in the drawing, even in the case where the insertion-type culture container is not used, the present biological sample packaging container can be used.

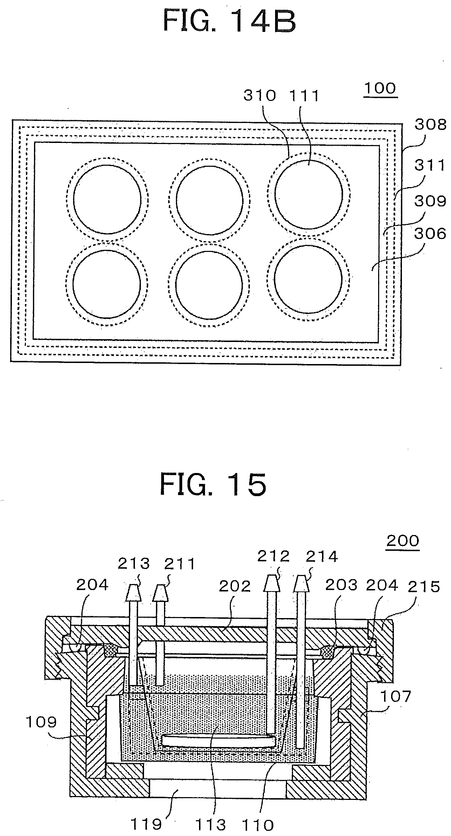

Next, FIG. 14A shows an example of a biological sample packaging container 100 packaging a 6-well plate 306, as an example in which a sample container of a different type is packaged. The cross-section of a cylindrical sample container or a container for an insertion-type culture container has a circular shape in the horizontal direction, and thus the container can be integrated with the packaging container main body part by rotating the packaging container lid part. On the other hand, the 6-well plate has a cuboidal shape. Accordingly, the drawing shows a case in which four corners of the packaging container 100 are fixed to be integrated using screws 307. As another method, it is obvious that the 6-well plate can be held while fixing plural positions by fitting, pins, or spring members. The 6-well plate 306 is packaged by a packaging container lid part 308 and a packaging container main body part 309. As similar to FIG. 1A, a leakage of the culture medium is prevented by a first gasket 310 and a second gasket 311 existing between the both. It should be noted that the 6-well plate 306 may be packaged in a state where an insertion-type culture container is put in the 6-well plate. Further, in order to conform the optical conditions when the 6-well plate is observed to those when only the 6-well plate is observed, holes for observation may be provided to the packaging container main body part 309. In this case, an elastic member is installed as similar to FIG. 1A to crimp between the 6-well plate and the packaging container main body part 309. A culture container 200 used for the automatic culture apparatus is configured in the same manner as the biological sample packaging container 100 except the configuration of the packaging container lid part 308.

As described above, even in the case of using the 6-well plate, the same effect as described above can be obtained.

In the culture container 200 used for the automatic culture apparatus in the first embodiment, the center O2 of the culture dish is shifted from the center O1 of the insertion-type culture container in order to easily secure a space where the flow channel tubes are inserted. In a fourth embodiment shown in FIG. 15, the center O2 of the culture dish of the culture container 200 is conformed to the center O1 of the insertion-type culture container on the premise of a manual culture process. In the case where a manual culture process is performed instead of automatic culture and the container is conveyed thereafter, the both centers may be conformed to each other as in the embodiment, as the sample storage container and the packaging container lid part.
100: BIOLOGICAL SAMPLE PACKAGING CONTAINER
101: SECOND PACKAGING CONTAINER LID PART
102,202: PACKAGING CONTAINER LID PART MAIN BODY
103: FIRST GASKET
104: SECOND GASKET
105: PACKAGING CONTAINER MAIN BODY PART
106: ELASTIC MEMBER
107: PACKAGING CONTAINER MAIN BODY PART BOTTOM SECTION
108: SCREW STRUCTURE
109: PACKAGING CONTAINER MAIN BODY PART HOLDING SECTION
110: SAMPLE CONTAINER (CULTURE CONTAINER)
111: CULTURE DISH
112: REGENERATED TISSURE
113: INSERTION-TYPE CULTURE CONTAINER
119: OBSERVATION HOLE
200: BIOLOGICAL SAMPLE PACKAGING CONTAINER
201: FIRST PACKAGING CONTAINER LID PART
211 - 214: FLOW CHANNEL TUBE
301: DENT PART
302,305: PACKAGING CONTAINER MAIN BODY PART BOTTOM SECTION
303,308: PACKAGING CONTAINER LID PART
304: GAS PERMEABLE MEMBRANE
306: 6-WELL PLATE
307: SCREW
309: PACKAGING CONTAINER MAIN BODY PART
310: FIRST GASKET
311: SECOND GASKET
501: CELL CONVEYANCE CONTAINER MAIN BODY
502: CELL CONVEYANCE CONTAINER LID
503: HEAT INSULATION MATERIAL
504: HEAT STORAGE MATERIAL BOX
505: CONTAINER STORAGE PART
506: CONTAINER
507: MONITORING DEVICE
601: HEAT STORAGE MATERIAL BOX
602: HEAT STORAGE MATERIAL
603: LINEAR MATERIAL
1000: CONTROLLER

## Claims

1. A biological sample packaging container (100,200) comprising:
a sample storage container (105) having a recessed part;
a lid member (102,202) that is adapted to seal the upper surface of the sample storage container; and
at least one gasket (103,104,203,204) that is provided on the rear surface of the lid member or on the upper surface of the sample storage container,
wherein the sample storage container is adapted for holding a biological sample (112) therein; **characterized in that**
the lid member includes a first lid member (202) provided with flow channel tubes (211-214) that allow liquid and gas to be moved between the sample storage container and the outside, and a second lid (102) having no flow channel tubes, and
the sample storage container is configured in such a manner that the first lid member and the second lid member can be replaced by each other.

2. The biological sample packaging container according to claim 1, wherein the gasket includes a first gasket (103,203) that is provided on the rear surface of the lid member or on the upper surface of the sample storage container and a second gasket (104,204) that is provided on the rear surface of the lid member or on the upper surface of the sample storage container at a position on the outer side relative to the first gasket, and the length of the first gasket (103,203) is longer than that of the second gasket (104,204) in the direction where the lid member is sealed to the sample storage container.

3. The biological sample packaging container according to claim 2, wherein:
in a state where the lid member is fixed to the sample storage container, the first gasket (103,203) has a function of sealing the upper surface of the recessed part and the second gasket has a function of sealing the upper surface of the sample storage container; and
when the lid member is sealed to the sample storage container, a tip end of the second gasket abuts on and is crimped to the opposed sample storage container or the lid member earlier than a tip end of the first gasket does.

4. The biological sample packaging container according to claim 1,
wherein a sample container of a lower layer (110) for storing the biological sample and an insertion-type culture container of an upper layer (113) are held in the sample storage container;
the sample container and the insertion-type culture container are open-system culture containers in each of which breathability between the inside and outside of the container is secured, and
in a state where the first or second lid member is fixed to the sample storage container, the first gasket seals a flange part of the insertion-type culture container held on the upper-end surface of the recessed part.

5. The biological sample packaging container according to claim 3,
wherein a sample container of a lower layer (110) for storing the biological sample and an insertion-type culture container of an upper layer (113) are held in the sample container,
the first (103,203) has a function of permitting the movement of gas to/from the sample container and the insertion-type culture container and of suppressing the movement of a culture medium when being not crimped; and
the first gasket and the second gasket have a function of suppressing the movement of gas and liquid to/from the sample container and the insertion-type culture container in the crimped state.

6. The biological sample packaging container according to claim 5, wherein as the flow channel tubes, the first lid member (202) is provided with a supply flow channel tube (211) and a discharge flow channel tube (212) used for the insertion-type culture container (113), and a supply flow channel tube (213) and a discharge flow channel tube (214) of the lower layer used for the sample container in the vertical direction.

7. The biological sample packaging container according to claim 3,
wherein the sample container of the lower layer (110) for storing the biological sample is held in the sample container;
the first gasket has a function of permitting the movement of gas to/from the sample container and of suppressing the movement of the culture medium when being not crimped;
the first gasket and the second gasket have a function of suppressing the movement of gas and liquid to/from the sample container in the crimped state, and
as the flow channel tubes, the first lid member is provided with the supply flow channel tube and the discharge flow channel tube of the lower layer used for the sample container in the vertical direction.

8. The biological sample packaging container according to claim 1,
wherein the lid member has a dent part (301) in a shape of protruding in the direction of the bottom surface of the sample storage container; and
in the case where the culture medium is supplied into the sample container up to a height exceeding the distance between the bottom surface of the dent part and the bottom surface of the sample storage container when the sample storage container and the lid member are sealed, the lid member is configured in such a manner that the culture medium and the bottom surface of the dent part are brought into contact with each other, an air space is formed between the bottom surface of the lid member other than the bottom surface of the dent part and the sample storage container, a stirring flow occurring in the culture medium by oscillation at the time of conveyance is accordingly suppressed from being generated, and shear stress is suppressed from being generated in cells.

9. A biological sample conveyance method using a biological sample packaging container (100,200), wherein the biological sample packaging container comprises a sample storage container (105) having a recessed part, a lid member (102,202) that is adapted to seal the upper surface of the sample storage container, and at least one gasket (103,104,203,204) that is provided on the rear surface of the lid member or on the upper surface of the sample storage container;
the lid member includes a first lid member (202) having flow channel tubes (211-214) used at the time of culture of the biological sample and a second lid member (102) having no flow channel tubes used at the time of conveyance of the biological sample;
the first lid member is removed to be replaced by the second lid member in a state where the biological sample while being held in the biological sample packaging container has been cultured using the sample storage container sealed with the first lid and
the biological sample is conveyed while being held in the biological sample packaging container using the sample storage container sealed with the second lid member.

10. The biological sample conveyance method using a biological sample packaging container according to claim 9,
wherein the sample storage container is filled with a culture medium for conveyance, after the first lid member (202) has been removed and before the sample storage container is covered with the second lid member (102) to form the sample storage container for conveyance; and the biological sample is conveyed.

## Patentansprüche

1. Verpackungsbehälter (100, 200) für biologische Proben, der Folgendes umfasst:
einen Probenaufbewahrungsbehälter (105), der ein vertieftes Teil aufweist;
ein Deckelelement (102, 202), das so ausgelegt ist, dass es die obere Oberfläche des Probenaufbewahrungsbehälters abdichtet; und
wenigstens eine Dichtung (103, 104, 203, 204), die an der unteren Oberfläche des Deckelelements oder an der oberen Oberfläche des Probenaufbewahrungsbehälters vorgesehen ist,
wobei der Probenaufbewahrungsbehälter ausgelegt ist, eine biologische Probe (112) aufzubewahren; **dadurch gekennzeichnet, dass**
das Deckelelement ein erstes Deckelelement (202), das mit Strömungskanalröhrchen (211-214) versehen ist, die ermöglichen, dass Flüssigkeit und Gas zwischen dem Probenaufbewahrungsbehälter und der Außenseite bewegt werden können, und ein zweites Deckelelement (102), das keine Strömungskanalröhrchen aufweist, umfasst, und
der Probenaufbewahrungsbehälter so konfiguriert ist, dass das erste und das zweite Deckelelement jeweils durch das andere ersetzt werden können.

2. Verpackungsbehälter für biologische Proben nach Anspruch 1, wobei die Dichtung eine erste Dichtung (103, 203), die an der unteren Oberfläche des Deckelelements oder an der oberen Oberfläche des Probenaufbewahrungsbehälters vorgesehen ist, und eine zweite Dichtung (104, 204), die an der unteren Oberfläche des Deckelelements oder an der oberen Oberfläche des Probenaufbewahrungsbehälters an einer Position an der Außenseite in Bezug auf die erste Dichtung vorgesehen ist, umfasst, und wobei die Länge der ersten Dichtung (103, 203) in der Richtung, in der das Deckelelement mit dem Probenaufbewahrungsbehälter abdichtet, größer als jene der zweiten Dichtung (104, 204) ist.

3. Verpackungsbehälter für biologische Proben nach Anspruch 2, wobei:
in einem Zustand, in dem das Deckelelement an dem Probenaufbewahrungsbehälter befestigt ist, die erste Dichtung (103, 203) eine Funktion zum Abdichten der oberen Oberfläche des vertieften Teils hat und die zweite Dichtung eine Funktion zum Abdichten der oberen Oberfläche des Probenaufbewahrungsbehälters hat; und
dann, wenn das Deckelelement mit dem Probenaufbewahrungsbehälter abdichtet, ein oberes Ende der zweiten Dichtung an dem gegenüberliegenden Probenaufbewahrungsbehälter anliegt und an diesen gequetscht wird, oder das Deckelelement anstelle eines oberen Endes der ersten Dichtung dafür verwendet werden kann.

4. Verpackungsbehälter für biologische Proben nach Anspruch 1,
wobei ein Probenbehälter einer unteren Lage (110) zum Aufbewahren der biologischen Probe und ein Kulturbehälter des Einsetztyps einer oberen Lage (113) in dem Probenaufbewahrungsbehälter gehalten werden;
der Probenbehälter und der Kulturbehälter des Einsetztyps Kulturbehälter mit offenem System sind, in denen eine Atmungsaktivität zwischen dem Innenraum und dem Außenraum des Behälters gewährleistet ist, und
in einem Zustand, in dem das erste oder das zweite Deckelelement an dem Probenaufbewahrungsbehälter befestigt ist, die erste Dichtung ein Flanschteil des Kulturbehälters des Einsetztyps, das an der Oberfläche am oberen Ende des vertieften Teils gehalten wird, abdichtet.

5. Verpackungsbehälter für biologische Proben nach Anspruch 3,
wobei ein Probenbehälter einer unteren Lage (110) zum Aufbewahren der biologischen Probe und ein Kulturbehälter des Einsetztyps einer oberen Lage (113) in dem Probenbehälter gehalten werden,
die erste Dichtung (103, 203) eine Funktion hat, dass sie die Bewegung von Gas zu bzw. von dem Probenbehälter und dem Kulturbehälter des Einsetztyps zulässt und die Bewegung eines Kulturmediums unterdrückt, wenn sie nicht gequetscht ist; und
die erste Dichtung und die zweite Dichtung eine Funktion haben, dass sie die Bewegung von Gas und Flüssigkeit zu bzw. von dem Probenbehälter und dem Kulturbehälter des Einsetztyps in dem gequetschten Zustand unterdrücken.

6. Verpackungsbehälter für biologische Proben nach Anspruch 5,
wobei für die Strömungskanalröhrchen das erste Deckelelement (202) mit einem Zufuhrströmungskanalröhrchen (211) und einem Austrittsströmungskanalröhrchen (212), die für den Kulturbehälter (113) des Einsetztyps verwendet werden, und mit einem Zufuhrströmungskanalröhrchen (213) und einem Austrittsströmungskanalröhrchen (214) der unteren Lage, die für den Probenbehälter verwendet werden, in der vertikalen Richtung versehen ist.

7. Verpackungsbehälter für biologische Proben nach Anspruch 3,
wobei der Probenbehälter der unteren Lage (110) zum Aufbewahren der biologischen Probe in dem Probenbehälter gehalten wird;
die erste Dichtung eine Funktion hat, dass sie die Bewegung von Gas zu bzw. von dem Probenbehälter zulässt und die Bewegung des Kulturmediums unterdrückt, wenn sie nicht gequetscht ist;
die erste Dichtung und die zweite Dichtung eine Funktion haben, dass sie die Bewegung von Gas und Flüssigkeit zu bzw. von dem Probenbehälter in dem gequetschten Zustand unterdrücken, und
für die Strömungskanalröhrchen das erste Deckelelement mit dem Zufuhrströmungskanalröhrchen und dem Austrittsströmungskanalröhrchen der unteren Lage, die für den Probenbehälter verwendet werden, in der vertikalen Richtung versehen ist.

8. Verpackungsbehälter für biologische Proben nach Anspruch 1,
wobei das Deckelelement ein vertieftes Teil (301) in einer Form aufweist, die in der Richtung der Bodenfläche des Probenaufbewahrungsbehälters vorsteht; und
in dem Fall, in dem das Kulturmedium dem Probenbehälter bis zu einer Höhe zugeführt wird, die den Abstand zwischen der Bodenfläche des vertieften Teils und der Bodenfläche des Probenaufbewahrungsbehälters, wenn der Probenaufbewahrungsbehälter und das Deckelelement abdichten, überschreitet, das Deckelelement so konfiguriert ist, dass das Kulturmedium und die Bodenfläche des vertieften Teils miteinander in Kontakt gebracht werden, ein Luftraum zwischen der Bodenfläche des Deckelelements, abgesehen von der Bodenfläche des vertieften Teils, und dem Probenaufbewahrungsbehälter ausgebildet wird, entsprechend unterdrückt wird, dass eine Wirbelströmung, die in dem Kulturmedium durch Schwingung zum Zeitpunkt des Transports eintreten würde, erzeugt wird, und unterdrückt wird, dass in Zellen eine Scherspannung erzeugt wird.

9. Transportverfahren für biologische Proben unter Verwendung eines Verpackungsbehälters (100, 200) für biologische Proben, wobei der Verpackungsbehälter für biologische Proben einen Probenaufbewahrungsbehälter (105), der ein vertieftes Teil aufweist, ein Deckelelement (102, 202), das so ausgelegt ist, dass es die obere Oberfläche des Probenaufbewahrungsbehälters abdichtet, und wenigstens eine Dichtung (103, 104, 203, 204), die an der unteren Oberfläche des Deckelelements oder an der oberen Oberfläche des Probenaufbewahrungsbehälters vorgesehen ist, umfasst;
wobei das Deckelelement ein erstes Deckelelement (202), das Strömungskanalröhrchen (211-214) aufweist, das zum Zeitpunkt der Züchtung der biologischen Probe verwendet wird, und ein zweites Deckelelement (102), das keine Strömungskanalröhrchen aufweist, das zum Zeitpunkt des Transports der biologischen Probe verwendet wird, umfasst,
wobei das erste Deckelelement entfernt wird, um in einem Zustand, in dem die biologische Probe, während sie in dem Verpackungsbehälter für biologische Proben gehalten wird, unter Verwendung des Probenaufbewahrungsbehälters, der mit dem ersten Deckel abdichtet, gezüchtet wird, durch das zweite Deckelelement ersetzt zu werden, und
die biologische Probe transportiert wird, wobei sie unter Verwendung des Probenaufbewahrungsbehälters, der mit dem zweiten Deckelelement abdichtet, in dem Verpackungsbehälter für biologische Proben gehalten wird.

10. Transportverfahren für eine biologische Probe unter Verwendung eines Verpackungsbehälters für eine biologische Probe nach Anspruch 9, wobei der Probenaufbewahrungsbehälter mit einem Kulturmedium zum Transport gefüllt wird, nachdem das erste Deckelelement (202) entfernt worden ist und bevor der Probenaufbewahrungsbehälter mit dem zweiten Deckelelement (102) bedeckt wird, um den Probenaufbewahrungsbehälter für den Transport zu bilden; und wobei die biologische Probe transportiert wird.

## Revendications

1. Conteneur d'emballage d'échantillons biologiques (100, 200) comprenant :
un conteneur de stockage d'échantillons (105) ayant une partie renfoncée ;
un élément de couvercle (102, 202) qui est adapté pour étancher la surface supérieure du conteneur de stockage d'échantillons ; et
au moins un joint (103, 104, 203, 204) qui est prévu sur la surface arrière de l'élément de couvercle ou sur la surface supérieure du conteneur de stockage d'échantillons,
dans lequel le conteneur de stockage d'échantillons est adapté pour contenir un échantillon biologique (112) à l'intérieur ;
**caractérisé en ce que** l'élément de couvercle inclut un premier élément de couvercle (202) doté de tubes formant canal d'écoulement (211-214) qui permettent à un liquide et à un gaz d'être déplacé entre le conteneur de stockage d'échantillons et l'extérieur, et un deuxième couvercle (102) n'ayant pas de tubes formant canal d'écoulement, et
le conteneur de stockage d'échantillons est configuré de telle manière que le premier élément de couvercle et le deuxième élément de couvercle peuvent être remplacés l'un par l'autre.

2. Conteneur d'emballage d'échantillons biologiques selon la revendication 1, dans lequel le joint inclut un premier joint (103, 203) qui est prévu sur la surface arrière de l'élément de couvercle ou sur la surface supérieure du conteneur de stockage d'échantillons, et un deuxième joint (104, 204) qui est prévu sur la surface arrière de l'élément de couvercle ou sur la surface supérieure du conteneur de stockage d'échantillons à une position sur le côté extérieur relativement au premier joint, et la longueur du premier joint (103, 203) est plus longue que celle du deuxième joint (104, 204) dans la direction dans laquelle l'élément de couvercle est scellé au conteneur de stockage d'échantillons.

3. Conteneur d'emballage d'échantillons biologiques selon la revendication 2, dans lequel :
dans un état dans lequel l'élément de couvercle est fixé au conteneur de stockage d'échantillons, le premier joint (103, 203) a une fonction de scellement de la surface supérieure de la partie renfoncée et le deuxième joint a une fonction de scellement de la surface supérieure du conteneur de stockage d'échantillons ; et
quand l'élément de couvercle est scellé au conteneur de stockage d'échantillons, une extrémité d'embout du deuxième joint vient en butée sur et est sertie au conteneur de stockage d'échantillons opposé ou à l'élément de couvercle plus tôt que ne le fait une extrémité d'embout du premier joint.

4. Conteneur d'emballage d'échantillons biologiques selon la revendication 1,
dans lequel un conteneur d'échantillons d'une couche inférieure (110) pour stocker l'échantillon biologique et un conteneur de culture de type à insertion d'une couche supérieure (113) sont contenus dans le conteneur de stockage d'échantillons ;
le conteneur d'échantillons et le conteneur de culture de type à insertion sont des conteneurs de culture à système ouvert dans chacun desquels la respirabilité entre l'intérieur et l'extérieur du conteneur est assurée, et
dans un état dans lequel le premier ou le deuxième élément de couvercle est fixé au conteneur de stockage d'échantillons,
le premier joint scelle une partie formant bride du conteneur de culture de type à insertion maintenue sur la surface d'extrémité supérieure de la partie renfoncée.

5. Conteneur d'emballage d'échantillons biologiques selon la revendication 3,
dans lequel un conteneur d'échantillons d'une couche inférieure (110) pour stocker l'échantillon biologique et un conteneur de culture de type à insertion d'une couche supérieure (113) sont contenus dans le conteneur d'échantillons,
le premier joint (103, 203) a pour fonction de permettre le déplacement de gaz vers/depuis le conteneur d'échantillons et le conteneur de culture de type à insertion, et de supprimer le déplacement d'un support de culture quand il n'a pas été serti ; et
le premier joint et le deuxième joint ont pour fonction de supprimer le déplacement de gaz et de liquide vers/depuis le conteneur d'échantillons et le conteneur de culture de type à insertion dans l'état serti.

6. Conteneur d'emballage d'échantillons biologiques selon la revendication 5, dans lequel, à titre de tubes formant canal d'écoulement, le premier élément de couvercle (202) est doté d'un tube formant canal d'écoulement d'alimentation (211) et d'un tube formant canal d'écoulement d'évacuation (212) utilisés pour le conteneur de culture de type à insertion (113), et d'un tube formant canal d'écoulement d'alimentation (213) et d'un tube formant canal d'écoulement d'évacuation (214) de la couche inférieure utilisés pour le conteneur d'échantillons dans la direction verticale.

7. Conteneur d'emballage d'échantillons biologiques selon la revendication 3,
dans lequel le conteneur d'échantillons de la couche inférieure (110) pour stocker l'échantillon biologique est contenu dans le conteneur d'échantillons ;
le premier joint a pour fonction de permettre le déplacement de gaz vers/depuis le conteneur d'échantillons et de supprimer le déplacement du support de culture quand il n'a pas été serti ;
le premier joint et le deuxième joint ont pour fonction de supprimer le déplacement de gaz et de liquide vers/depuis le conteneur d'échantillons dans l'état serti, et
à titre de tubes formant canal d'écoulement, le premier élément de couvercle est doté du tube formant canal d'écoulement d'alimentation et du tube formant canal d'écoulement d'évacuation de la couche inférieure utilisés pour le conteneur d'échantillons dans la direction verticale.

8. Conteneur d'emballage d'échantillons biologiques selon la revendication 1,
dans lequel l'élément de couvercle a une partie crantée (301) en forme de projection dans la direction de la surface de fond du conteneur de stockage d'échantillons ; et
dans le cas où le support de culture est fourni jusque dans le conteneur d'échantillons jusqu'à une hauteur excédant la distance entre la surface de fond de la partie crantée et la surface de fond du conteneur de stockage d'échantillons quand le conteneur de stockage d'échantillons et l'élément de couvercle sont scellés, l'élément de couvercle est configuré de telle manière que le support de culture et la surface de fond de la partie crantée sont amenés en contact l'un avec l'autre, un espace d'air est formé entre la surface de fond de l'élément de couvercle autre que la surface de fond de la partie crantée et du conteneur de stockage d'échantillons, la génération d'un écoulement de brassage qui se produit dans le support de culture par oscillation au moment du transport est ainsi supprimée, et la génération d'une contrainte de un cisaillement dans des cellules est ainsi supprimée.

9. Procédé de transport d'échantillons biologiques utilisant un conteneur d'emballage d'échantillons biologiques (100, 200),
dans lequel le conteneur d'emballage d'échantillons biologiques comprend un conteneur d'emballage d'échantillons (105) ayant une partie renfoncée, un élément de couvercle (102, 202) qui est adapté pour sceller la surface supérieure du conteneur de stockage d'échantillons, et au moins un joint (103, 104, 203, 204) qui est prévu sur la surface arrière de l'élément de couvercle ou sur la surface supérieure du conteneur de stockage d'échantillons ;
l'élément de couvercle inclut un premier élément de couvercle (202) ayant des tubes formant canal d'écoulement (211-214), utilisé au moment d'une culture de l'échantillon biologique, et un deuxième élément de couvercle (102) n'ayant pas de tubes formant canal d'écoulement, utilisé au moment du transport de l'échantillon biologique ;
le premier élément de couvercle est enlevé pour être remplacé par le deuxième élément de couvercle dans un état dans lequel l'échantillon biologique, pendant qu'il est contenu dans le conteneur d'emballage d'échantillons biologiques, a été cultivé en utilisant le conteneur de stockage d'échantillons scellé avec le premier couvercle, et
l'échantillon biologique est transporté pendant qu'il est contenu dans le conteneur d'emballage d'échantillons biologiques en utilisant le conteneur de stockage d'échantillons scellé avec le deuxième élément de couvercle.

10. Procédé de transport d'échantillons biologiques utilisant un conteneur d'emballage d'échantillons biologiques selon la revendication 9,
dans lequel on remplit le conteneur de stockage d'échantillons d'un support de culture pour le transport après que le premier élément de couvercle (202) a été enlevé et avant que le conteneur de stockage d'échantillons soit recouvert avec le deuxième élément de couvercle (102) pour former le conteneur de stockage d'échantillons pour le transport ; et on transporte l'échantillon biologique.
